# EUROPEAN PATENT SPECIFICATION

(11) **EP 3 728 552 B1**
(45) Date of publication and mention of the grant of the patent: **26.05.2021**
(21) Application number: 18829865.7
(22) Date of filing: 21.12.2018
(51) Int. Cl.: C12M 3/00, B01L 3/00, C12M 3/06

(54) **MICROFLUIDIC DEVICES IMPLEMENTING BLOOD AND URINE CIRCUIT FOR EMULATING HOMEOSTATIC MICROPHYSIOLOGICAL SYSTEM**
MIKROFLUIDISCHE VORRICHTUNGEN ZUR DURCHFÜHRUNG EINES BLUT- UND URINKREISLAUFS ZUR EMULATION EINES HOMÖOSTATISCHEN MIKROPHYSIOLOGISCHEN SYSTEMS
DISPOSITIFS MICROFLUIDIQUES METTANT EN OEUVRE UN CIRCUIT DU SANG ET DE L'URINE POUR ÉMULER UN SYSTÈME MICROPHYSIOLOGIQUE HOMÉOSTATIQUE

(30) Priority: 22.12.2017 EP 17210385; 22.12.2017 US 201762609567 P
(43) Date of publication of application: 28.10.2020
(73) Proprietor: TissUse GmbH, 13347 Berlin (DE)
(72) Inventor: MARX, Uwe, 15528 Spreenhagen (DE); DEHNE, Eva-Maria, 14165 Berlin (DE); HASENBERG, Tobias, 1030 Bruxelles (BE)
(74) Representative: Grünecker Patent- und Rechtsanwälte PartG mbB
(86) International application number: PCT/EP2018/086628
(87) International publication number: WO 2019/122349

(56) References cited:
- WO-A1-2011/040889
- WO-A1-2013/086329
- WO-A1-2013/086486
- US-A1- 2011 024 346

## Description

### TECHNICAL FIELD

The present disclosure relates to novel microfluidic devices and their use for operating microphysiological systems. The novel microfluidic devices are particularly useful for pharmacokinetic-pharmacodynamic analysis in the context of ADME(T) profiling. The present disclosure provides novel microfluidic devices comprising a first "blood" circuit with one or more cell culture compartments, and a second "urine" circuit comprising a renal filtration and reabsorption unit, wherein both circuits are connected with each other via the filtration unit and the reabsorption unit. The present disclosure further provides methods, kits and assays implementing the novel microfluidic devices.

### TECHNICAL BACKGROUND

Microfluidic devices are capable of emulating human biology *in vitro* at the smallest biologically acceptable scale. In contrast to conventional (static) cell and tissue cultures, microphysiological systems apply dynamic fluid flow for physiological nutrition of the tissues as a factor for mimicking organ function with a minimum use of human cells and tissues. The technology of multi-organ-chips is of particular interest to the pharmaceutical industry in terms of drug development and toxicity testing. Multi organ-on-a-chip models can enhance the fundamental understanding of complex biological processes and are considered to bring about more rapid, accurate, cost-effective, and clinically relevant testing of drugs as well as cosmetics. There is thus a growing recognition in the art that *in vitro* testing using microphysiological systems implemented on microfluidic devices can be more effective and also be more reliable than *in vivo* testing.

There is a need in the art for novel multi-organ microfluidic systems that support physiological conditions for maintaining stable microenvironments over a long period of time, and that enable mimicking the physiological interaction of two or more models emulating organ functionality. The present disclosure addresses this need by providing novel microfluidic devices, which are specifically characterized by a blood circuit and a urine circuit establishing a homeostatic microphysiological system.

Sakolish and Mahler (RSC Adv., 2017, 7, 4216-4225) describe the layout of the renal filtration barriers of glomerulus and tubulus on a microfluidic device that is specifically used in the culturing of human kidney cells. Giobbe et al. (Nat. Methods, 2015, 12(7), 637-640) describe the functional differentiation of human pluripotent stem cells on a chip. Zhang et al. (Future Sci. OA, 2017, 3(2), FSO197) describe stem cell cultures and differentiation in microfluidic devices towards organ-on-a-chip. WO2013086329A describes a microfluidic device adapted to mimic tissues and organs. One of the organs to be emulated is the kidney, comprising two microfluidc channels and a membrane.

### SUMMARY OF THE INVENTION

The novel microfluidic device provided by the present disclosure allows, *inter alia,* emulating physiological interactions of two or more organ functionalities within one system to enable data generation from a homeostatic microphysiological system. The microphysiological system established with the novel microfluidic device supports physiological conditions for maintaining stable microenvironments in a system mimicking the interaction and crosstalk of a number of models emulating organ functionality.

The novel microfluidic device provided by the present disclosure enables emulation of human organ-to-organ crosstalk, ADME pathways and systemic regulatory circuits between organ models, as demonstrated herein. In particular, the novel microfluidic device provides for a microphysiological system with a stable microenvironment, particularly suitable for assay establishment for pharmacokinetic-pharmacodynamic analysis in the context of ADME(T) profiling.

The novel microfluidic devices provided by the present disclosure further provide for organoid or tissue engineering with microphysiological *in vitro* systems using induced pluripotent stem cell (iPSC)-derived organ-specific precursor cells or iPSC-derived organ-specific organoids.

In particular, the present disclosure provides:
[1] A microfluidic device comprising a blood circuit with one or more cell culture compartments and a urine circuit comprising a filtration unit and a reabsorption unit, wherein both circuits are connected with each other via the filtration unit and the reabsorption unit, further comprising a reservoir compartment in addition to the one or more cell culture compartments, which serves to add nutrient solution to the system.
[2] The microfluidic device of [1], wherein the filtration unit comprises a filtration barrier that selectively allows the passage of molecules from the first circuit to the second circuit based on size and charge of the molecules.
[3] The microfluidic device of [2], wherein the filtration barrier is a mechanical or biological barrier, preferably a biological barrier that comprises podocytes.
[4] The microfluidic device of any one of [1]-[3], wherein the reabsorption unit comprises a barrier that allows reabsorption of fluid from the second circuit to the first circuit.
[5] The microfluidic device of [4], wherein the reabsorption barrier is a biological barrier, preferably wherein the biological barrier comprises renal tubule cells.
[6] The microfluidic device of any one of [1]-[5], wherein at least one of the cell culture compartments of the first circuit comprises an organ equivalent mimicking liver function, and at least one of the cell culture compartments of the first circuit comprises an organ equivalent mimicking intestine function.
[7] Use of the microfluidic device of any one of [1]-[6] in analytical testing, diagnostics, research, target validation, toxicity studies, tissue engineering, tissue manufacturing, drug screening, and/or pharmacokinetic-pharmacodynamic analysis.
[8] A method of operating a microphysiological system making use of the microfluidic device of any one of [1]-[6].
[9] The method of [8], further comprising selectively adding a nutrient solution to the microphysiological system via the cell culture compartment comprising an organ equivalent or tissue comprising epithelial cells of the gastrointestinal tract and mimicking intestine function.
[10] A method of detecting an analyte in a microphysiological system making use of the microfluidic device of any one of [1]-[6], wherein the method comprises adding a test sample to a cell culture compartment of the first circuit, preferably wherein the cell culture compartment comprises a tissue comprising epithelial cells of the gastrointestinal tract, the skin, or the respiratory tract.
[11] A method of mimicking homeostasis comprising operating a microphysiological system comprising a first circuit with one or more cell culture compartments and a second circuit comprising a filtration unit and a reabsorption unit, wherein both circuits are connected with each other via the filtration unit and the reabsorption unit.
[12] The method of [11], comprising selectively adding a nutrient solution to the microphysiological system via a cell culture compartment comprising epithelial cells of the gastrointestinal tract, the skin, or the respiratory tract, preferably wherein the epithelial cells are epithelial cells of the gastrointestinal tract, preferably of the small intestine.
[13] A method of culturing and/or maintaining cells comprising seeding the microfluidic device of any one of claims [1]-[6] with cells.
[14] A kit for operating a microphysiological system comprising the microfluidic device of any one of claims [1]-[6] and instructions for use.
[15] An ADME (absorption, distribution, metabolism, excretion) assay, or an ADMET (absorption, distribution, metabolism, excretion, toxicity) assay, comprising the microfluidic device according to any one of claims [1]-[6].

**BRIEF DESCRIPTION OF THE DRAWINGS**

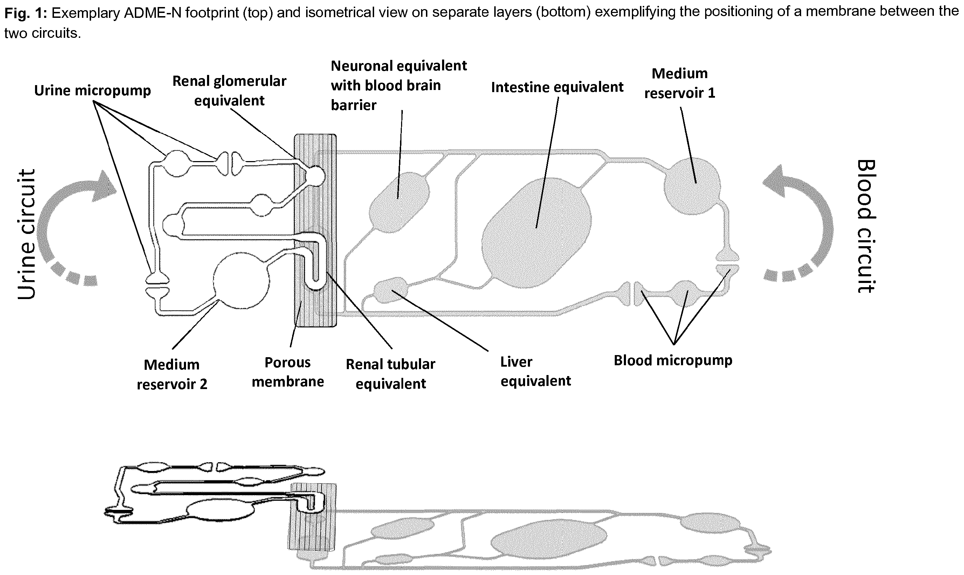

- Figure 1:: shows an exemplary ADME-N chip footprint (top) and isometrical view on separate layers (bottom). The isometrical view exemplifies the positioning of a membrane between the two circuits (blood circuit and urine circuit). ADME-N = ADME chip plus neuronal equivalent.

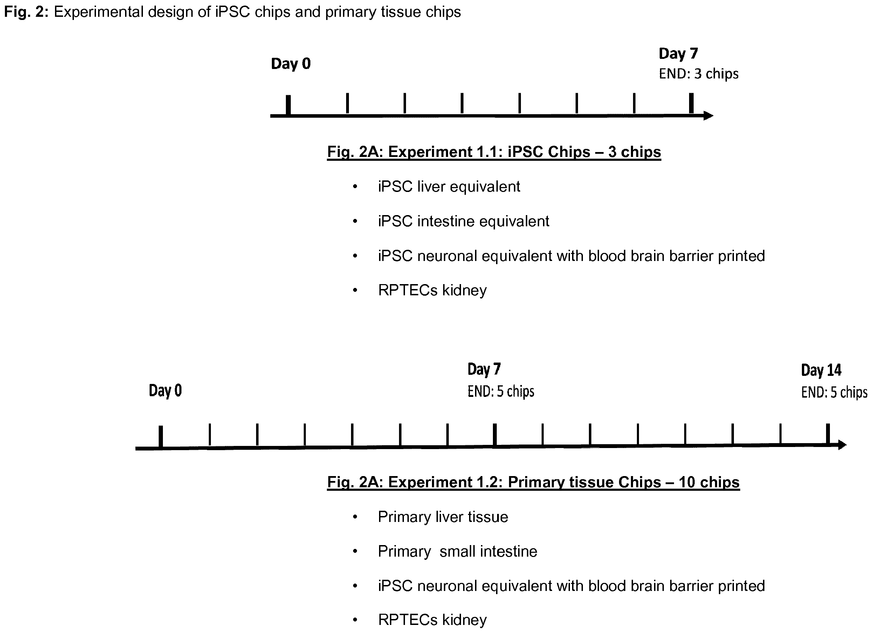

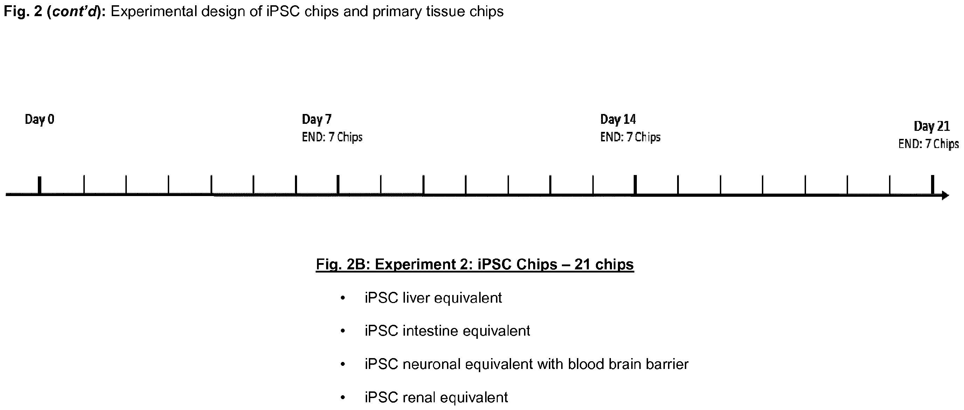

- Figure 2:: shows the experimental design of iPSC chips and primary tissue chips. RPTECs = renal proximal tubule epithelial cell line. iPSC = induced pluripotent stem cell. **2A:** Design of Experiments 1.1 and 1.2, **2B:** Design of Experiment 2.

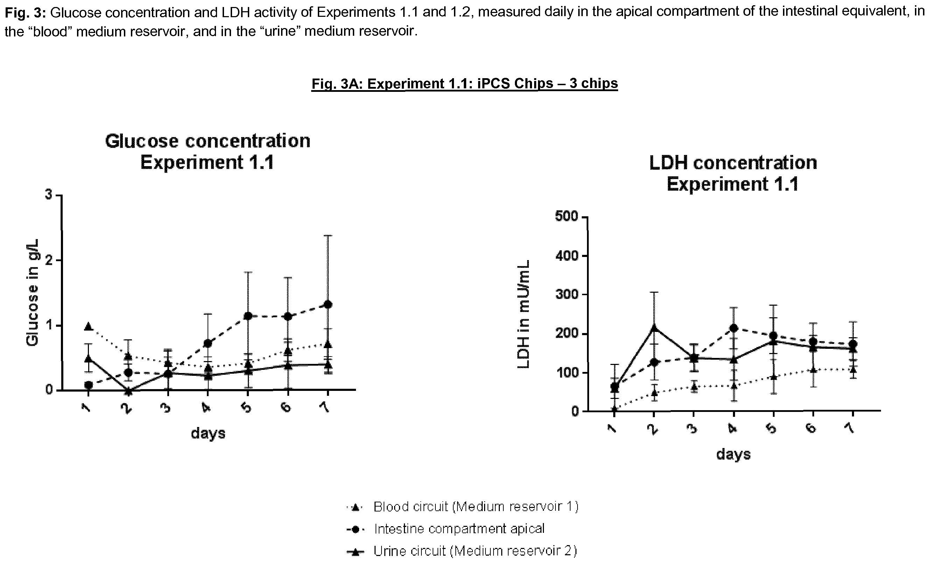

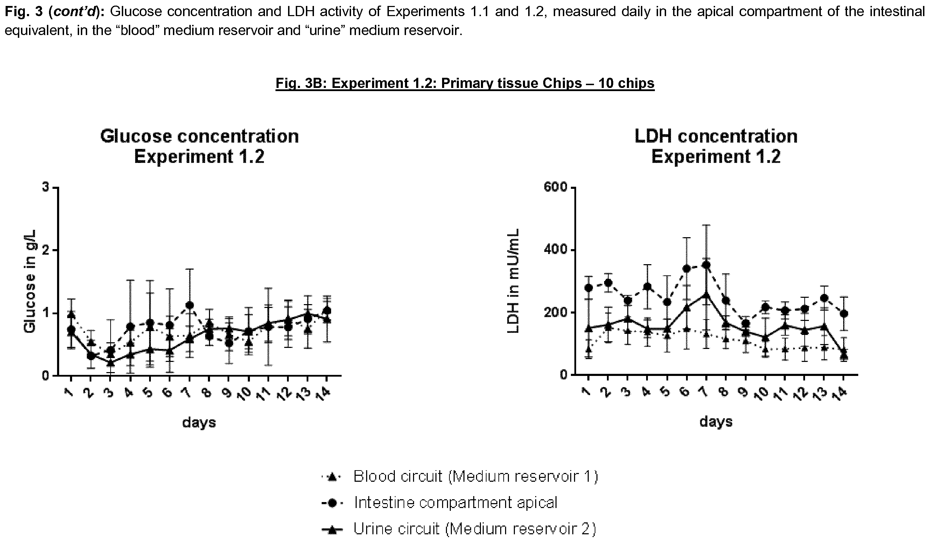

- Figure 3:: shows measurements of glucose concentration and LDH activity. **3A:** measurements of glucose concentration and LDH activity of Experiment 1.1 (iPSC chips); **3B:** measurements of glucose concentration and LDH activity of Experiment 1.2 (Primary tissue chips). Measurements were made daily in the apical compartment of the intestinal equivalent, in the "blood" medium reservoir (for the blood circuit), and in the "urine" medium reservoir (for the urine circuit).

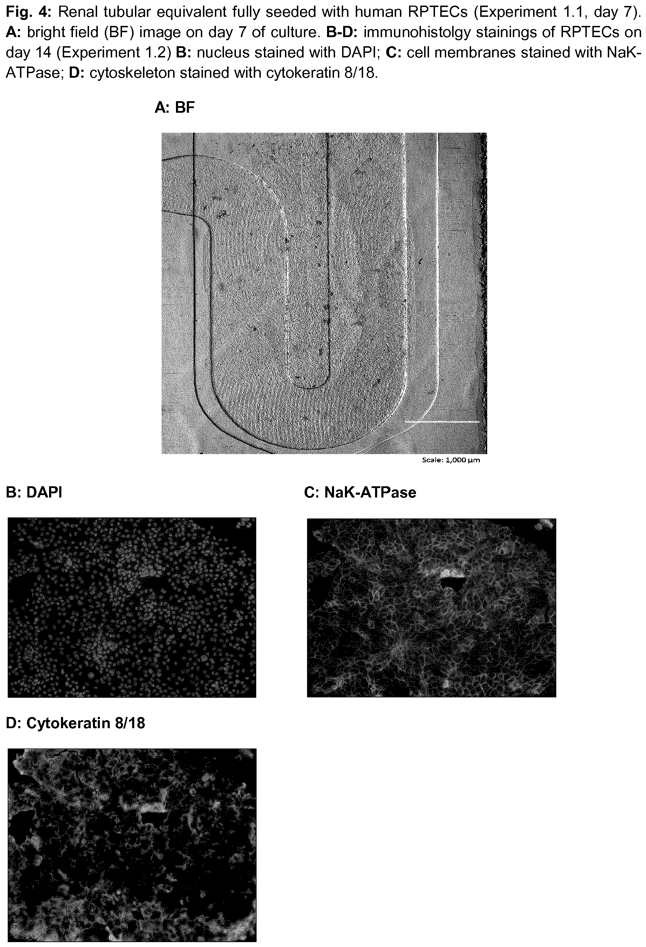

- Figure 4:: shows the renal tubular equivalent of the ADME-N chip fully seeded with human RPTECs (Experiment 1.1, day 7). **A:** bright field (BF) image on day 7 of culture. **B-D:** immunohistologic staining of RPTECs on day 14 (Experiment 1.2) **B**: nucleus stained with DAPI (4',6-Diamidin-2-phenylindol); **C:** cell membranes stained with NaK-ATPase; **D:** cytoskeleton stained with cytokeratin 8/18. RPTECs = renal proximal tubule epithelial cells.

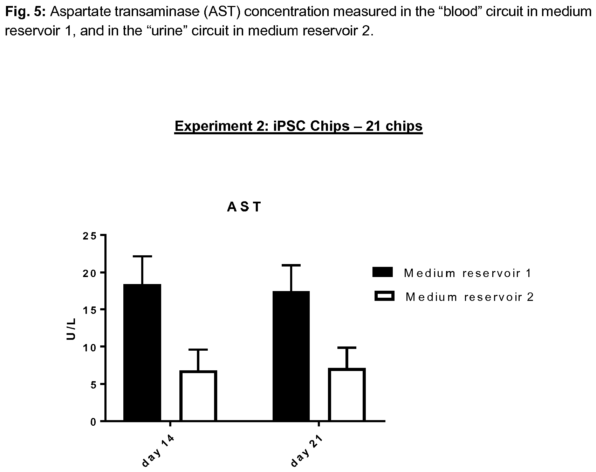

- Figure 5:: shows measurements of aspartate transaminase (AST) concentration in the "blood" circuit in medium reservoir 1, and the "urine" circuit in medium reservoir 2.

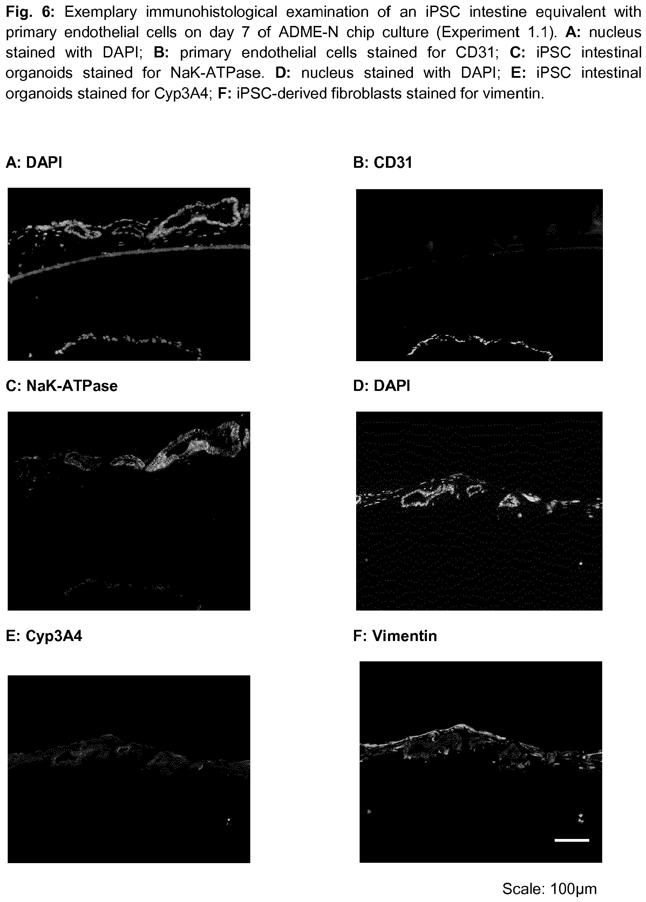

- Figure 6:: shows exemplary immunohistological examination of an iPSC intestine equivalent with primary endothelial cells on day 7 of the ADME-N chip culture (Experiment 1.1). **A:** nucleus stained with DAPI; **B**: primary endothelial cells stained for CD31; **C:** iPSC intestinal organoids stained for NaK-ATPase. **D:** nucleus stained with DAPI; **E:** iPSC intestinal organoids stained for Cyp3A4; **F:** iPSC-derived fibroblasts stained for vimentin.

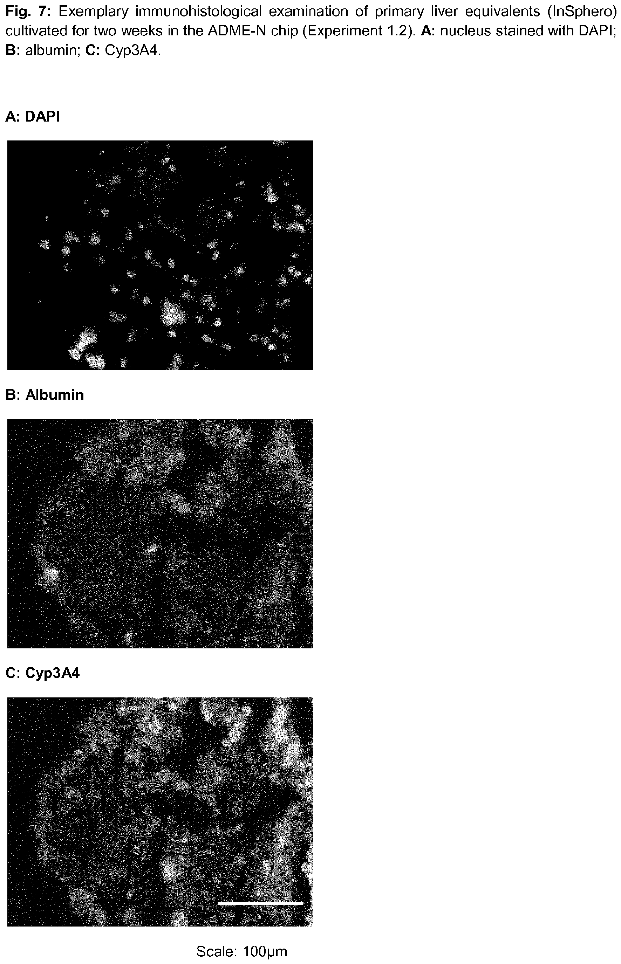

- Figure 7:: shows exemplary immunohistological examination of primary liver equivalents (InSphero) cultivated for two weeks in the ADME-N chip (Experiment 1.2). **A:** nucleus stained with DAPI; **B**: albumin; **C:** Cyp3A4.

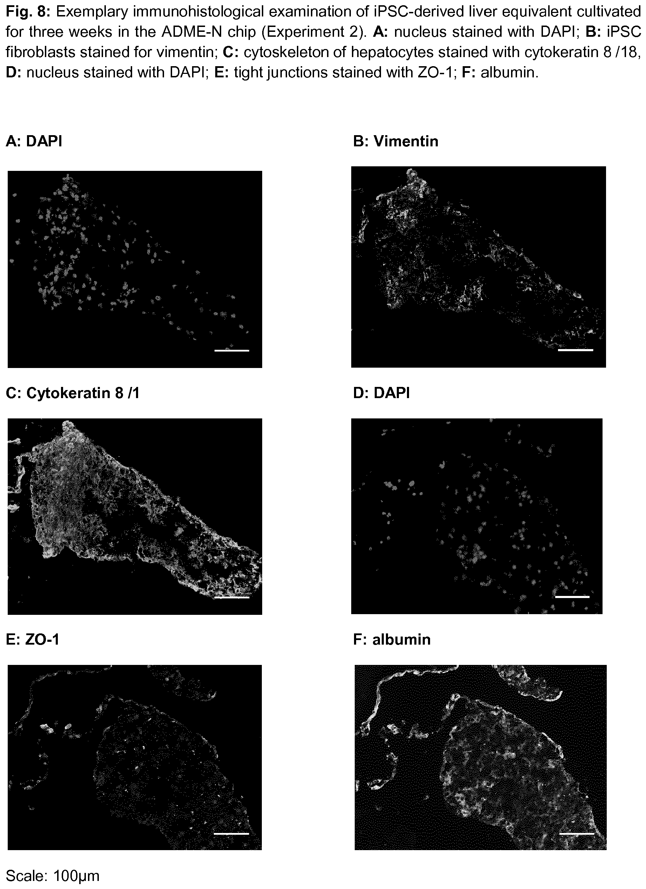

- Figure 8:: shows exemplary immunohistological examination of iPSC-derived liver equivalent cultivated for three weeks in the ADME-N chip (Experiment 2). **A:** nucleus stained with DAPI; **B**: iPSC fibroblasts stained for vimentin; **C:** cytoskeleton of hepatocytes stained with cytokeratin 8 /18, **D:** nucleus stained with DAPI; **E:** tight junctions stained with ZO-1; **F:** albumin.

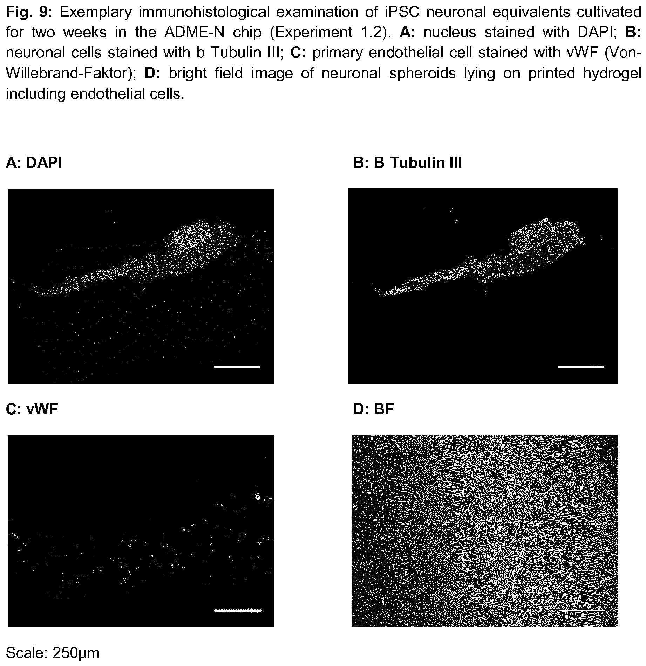

- Figure 9:: shows exemplary immunohistological examination of iPSC neuronal equivalents cultivated for two weeks in the ADME-N chip (Experiment 1.2). **A:** nucleus stained with DAPI; **B**: neuronal cells stained with b Tubulin III; **C:** primary endothelial cell stained with vWF (von-Willebrand-Faktor); **D:** bright field image of neuronal spheroids lying on printed hydrogel including endothelial cells.

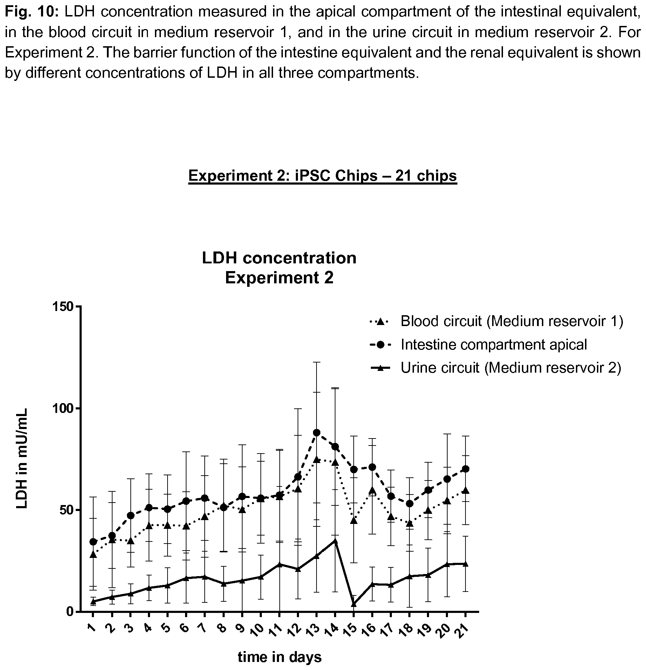

- Figure 10:: shows measurement of LDH concentration in the apical compartment of the intestinal equivalent, in the blood circuit in medium reservoir 1, and in the urine circuit in medium reservoir 2 (Experiment 2). The barrier function of the intestine equivalent and the renal equivalent is shown by different concentrations of LDH in all three compartments.

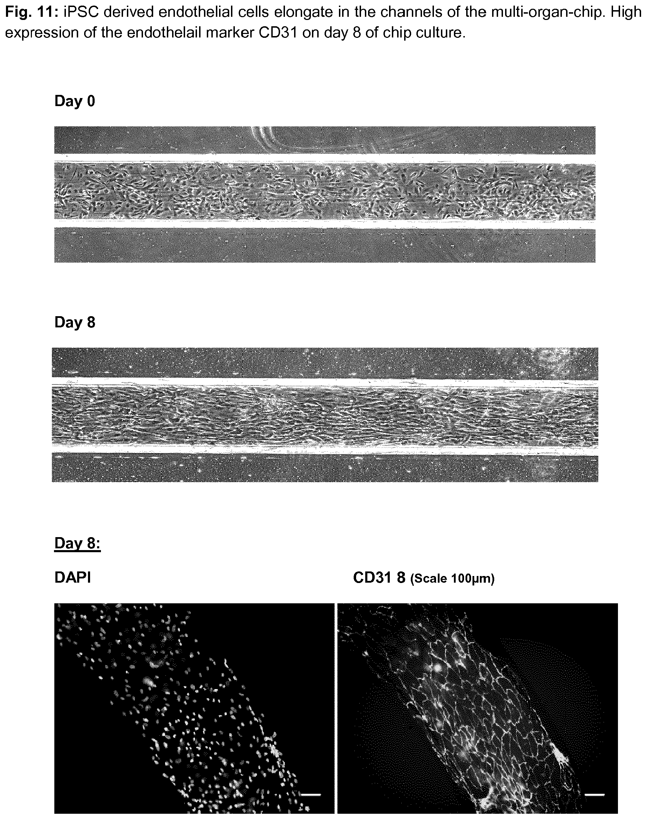

- Figure 11:: shows elongation of iPSC-derived endothelial cells in the channels of the multi-organ-chip. High expression of the endothelial marker CD31 on day 8 of chip culture.

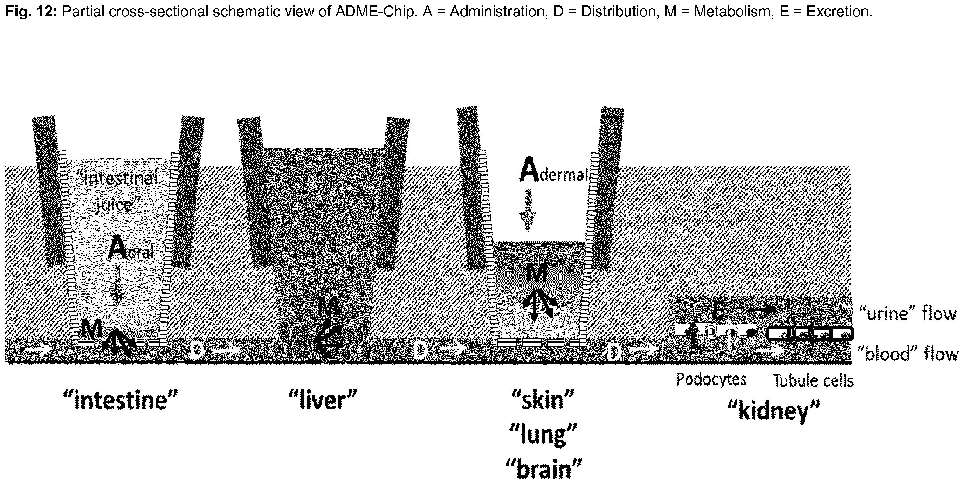

- Figure 12:: shows a cross-sectional schematic view of an ADME-Chip of the present disclosure. A = Administration, D = Distribution, M = Metabolism, E = Excretion.

### DETAILED DESCRIPTON OF THE INVENTION

The novel microfluidic device provided by the present disclosure allows emulating physiological interactions of two or more organ functionalities within one system to enable data generation from a homeostatic microphysiological system. The microphysiological system established with the novel microfluidic device supports physiological conditions mandatory to maintain stable microenvironments in a system mimicking the interaction and crosstalk of a number of models emulating organ functionality. Figure 1 shows an exemplary ADME-N (ADME + neuronal equivalent) chip footprint (top) and isometrical view on separate layers (bottom). The isometrical view exemplifies the positioning of a membrane between the two circuits (blood circuit and urine circuit). Fig. 12 shows a partial cross-sectional schematic view of an ADME chip of the present disclosure.

Fig. 2 shows the experimental design of iPSC chips and primary tissue chips. Sets of chips were cultivated for 7, 14 and 21 days. At the endpoints, cellular samples were taken for immunohistological examinations, qPCR and RNA sequencing. Supernatants were analysed for their glucose, lactate, LDH (lactate dehydrogenase), albumin, urea, ALT (alanine aminotransferase) and AST (aspartate transaminase) content. Controls from static cultivations of the given organ equivalents were taken on day 0, 7 and 14.

The novel microfluidic devices provided by the present disclosure specifically provide for organoid or tissue engineering with microphysiological *in vitro* systems using induced pluripotent stem cell (iPSC)-derived organ-specific precursor cells or iPSC-derived organ-specific organoids. The present disclosure also demonstrates that shear stress promotes elongation of iPSC-derived endothelial cells in the channels of the multi-organ-chip (Fig. 11).

The novel microfluidic device provided by the present disclosure enables emulation of human organ-to-organ crosstalk, ADME pathways and systemic regulatory circuits between organ models, as demonstrated herein. In particular, the novel microfluidic device provides for a microphysiological system with a stable microenvironment, particularly suitable for assay establishment for pharmacokinetic-pharmacodynamic analysis in the context of ADME(T) profiling. The novel microfluidic device provided by the present disclosure has been shown to establish and maintain homeostatic stability of the microphysiological system, as evidenced by the glucose and LDH measurements depicted in Figures 3 and 10.

The glomerular and tubular compartments (filtration unit and reabsorption unit) can be accomplished with a combination of a polycarbonate membrane and a tight layer of renal cells (Fig. 4). The former implements a technical barrier to filter larger molecular weight medium constituents and retards their diffusion. The latter reabsorbs substances and causes the build-up of concentration gradients across the "blood" and "urine" circuit. This gradient is visible not only for LDH, but also for aspartate transaminase (AST, Fig. 5). Both of their distinctly different concentrations demonstrate the function of the renal membrane of the novel microfluidic ADME chip provided by the present disclosure. The AST originates from the liver equivalent as it is an intracellular enzyme found in hepatocytes.
All organ equivalents were immunohistochemically assessed to determine identity, functionality, and viability (Figs. 6-9).

Metabolic analysis revealed the establishment of a reproducible homeostasis between all organ equivalents. The present disclosure enables the demonstration of cell polarization, reorganization, barrier function, and trafficking that is observed *in vivo.* The achievement of a stable microenvironment in the novel microfluidic device combining a first circuit with one or more cell culture compartments and a second circuit comprising a filtration and reabsorption unit overlapping with the first circuit in the renal units opens possibilities for repeated dose testing, e.g., in drug development.

The present disclosure provides a novel microfluidic device comprising a first circuit with one or more cell culture compartments, and a second circuit comprising a filtration unit and a reabsorption unit, wherein both circuits are connected with each other via the filtration unit and the reabsorption unit. Figure 1 shows an exemplary layout of a novel microfluidic device comprising two circuits (termed "blood circuit" and "urine circuit") containing cavities for the incorporation of the following organ equivalents: intestine, liver, kidney (segregated into glomerulus and tubulus), and neuronal tissue. The former three tissues are used to accomplish the so-called ADME profile (adsorption, distribution, metabolism, excretion). The latter is an additional tissue supplementing that profile. The chip is, thus, termed ADME-N chip (ADME + neuronal equivalent/tissue). One reservoir compartment in each circuit allows sampling of supernatants ("medium reservoir 1 and 2"). The medium is perfused through the microfluidic network by two incorporated, pneumatic micropumps - one for each circuit. The circuits overlap in the two renal unit compartments and are separated by a porous membrane made of polycarbonate.

As compared to single-organ systems/chips, the microphysiological system/ microfluidic chip of the present disclosure can be considered as a multi-organ system/chip given that the microfluidic device comprises two circuits, and thereby comprises at least two cell culture compartments, with one of them being localized in the first circuit, and the other being one of the units of the second circuit mimicking renal function, in particular the reabsorption unit.

In various embodiments, the novel microfluidic device may be considered as comprising a first circuit with one or more cell culture compartments, and a second circuit comprising a filtration unit and a reabsorption unit, wherein both circuits overlap in the filtration unit and the reabsorption unit. In various preferred embodiments, the overlapping parts of the two circuits (or the parts connecting both circuits with each other) are separated by a porous membrane. These overlapping/connecting parts specifically mean the filtration unit and the reabsorption unit.

In various embodiments, the said porous membrane separating the overlapping/connecting parts of the two circuits is a synthetic or a biological porous membrane. In various embodiments, the said porous membrane may be made of any of collagen, fibrin, extracellular matrix (including decellularized organ matrix), or mixtures thereof, without being limited thereto. In various embodiments, the porous membrane exhibits the same properties as a Transwell® insert. In various embodiments, the porous membrane is resorbable by cells. In various other embodiments, the porous membrane is made of polycarbonate. In various other embodiments, the porous membrane is a porous membrane made of poly(dimethylsiloxane) (PDMS).

The filtration unit and the reabsorption unit are separate cell culture compartments for incorporation of kidney cells, in particular a renal glomerular equivalent (filtration unit) and a renal tubular equivalent (reabsorption unit).

As described herein, the terms "cell culture compartment(s)" and "cell culture chamber(s)" or "cell culture cavity/ies" may be used interchangeably herein. A cell culture compartment according to the present disclosure is a compartment, chamber or cavity particularly suitable for culturing cells and tissues as used and described in the context of the present disclosure. In various embodiments, the cell culture compartment comprises a well plate format, in particular (micro)well cell culture plates (or multiwells) in any of 6-, 12-, 24-, 48-, 96-, and 384-well format. Preferred embodiments encompass multiwells in 24-, 48-, 96- or 384-well format. The multiwells can be made from a variety of materials, while the most common materials are polystyrene and polypropylene, which can be used in various embodiments of the present disclosure. In various other embodiments, the cell culture compartment is a Transwell® (microfluidic) insert, more preferably a Transwell® (microfluidic) insert made of polycarbonate (PC), polyester (PE), collagen-coated polytetrafluoroethylene (PTFE), or PET (polyethylene terephthalate). As described herein, a cell culture compartment used in the context of the present disclosure can be described to have a porous membrane (surface) for cell/tissue culture and growth.

As described herein, a porous membrane specifically means, without being limited thereto, a membrane having a porous structure that may be used not only for filtering, metering, and/or separating chemical and/or biological molecules, but also as a surface for cell or tissue culture and growth. The porous membrane of a cell culture compartment as used in the context of the present disclosure specifically serves to establish a fluid connection between the organ equivalent comprised by the cell culture compartment and the microfluidic network. Accordingly, in various embodiments, the porous membrane of a cell culture compartment as described herein is arranged such that the organ equivalent (cells/tissue) positioned on the surface of the porous membrane is in fluid connection with the microfluidic channels of the microfluidic device. In various embodiments, the porous membrane can be seeded with cells on both sides of the membrane. Preferably, endothelial cells are seeded at the bottom side of the porous membrane.
In general, through manufacturing processes the pore sizes can be tuned from a few nanometers to micrometers, thereby enabling the filtration, metering and separation of targeted chemical and biological molecules, and also the fluidic flow between the organ equivalents in the cell culture compartments and the microfluidic channel network. In various embodiments, the porous membrane is a porous membrane as described above. Thus, the porous membrane may be a synthetic or a biological porous membrane. In various embodiments, the said porous membrane may be made of any of collagen, fibrin, or extracellular matrix (including decellularized organ matrix) without being limited thereto. In various embodiments, the porous membrane exhibits the same properties as a Transwell® insert. In various embodiments, the porous membrane is resorbable by cells. In various other embodiments, the porous membrane is made of polycarbonate. In various other preferred embodiments, the porous membrane is a porous membrane made of poly(dimethylsiloxane (PDMS).

As described herein, the term "first circuit" specifically means a circulatory system mimicking a mammalian blood circulatory system, more specifically mimicking the human blood circulatory system. As further described herein, the terms "first circuit" and "blood circuit equivalent" or "biomimetic blood circuit" may be used interchangeably herein. Likewise, as described herein, the term "second circuit" specifically means a circulatory system mimicking a mammalian urinary system, more specifically mimicking the human urinary system. As further described herein, the terms "second circuit" and "urine circuit equivalent" or "biomimetic urine circuit" may be used interchangeably herein.

Renal filtration includes both passive and active filtration, with the glomerulus acting as passive filtration barrier preventing larger molecular weight serum proteins from entering the nephron. Accordingly, such larger molecular weight serum proteins continue circulating in the blood stream. As described herein, the filtration unit of the novel microfluidic device disclosed herein comprises a filtration barrier that selectively allows the passage of molecules from the first circuit ("blood circuit") to the second circuit ("urine circuit") based on size and charge of the molecules, thereby mimicking the selective glomerulus filtration barrier.

In various embodiments of the present disclosure, the filtration barrier comprised by the filtration unit is established by the porous membrane separating the filtration unit (and the reabsorption unit) from the blood circuit (separating the overlapping parts of the two circuits). Thus, according to such embodiments, the filtration barrier may be considered a (porous) mechanical filtration barrier. Specifically, the (porous) mechanical filtration barrier is mimicking the glomerulus filtration barrier. Accordingly, in various embodiments, the filtration barrier may even be established by a cell culture compartment having a porous membrane. In preferred embodiments, the mechanical filtration barrier has a pore size of approximately 8 nm, but may have a pore size ≤ 8 nm. According to other preferred embodiments, the mechanical filtration barrier has a pore size setting an upper limit of about 69 kDa for serum proteins to enter the second (urine) circuit. In various embodiments, the mechanical filtration barrier has a pore size setting a limit for albumin, preferably serum albumin (or blood albumin), to enter the second (urine) circuit.

In various other embodiments of the present disclosure, the filtration barrier comprised by the filtration unit is a biological filtration barrier. Preferably, the biological filtration barrier is a barrier having filtration slits setting an upper limit of about 69 kDa for serum proteins to enter the second (urine) circuit. In various embodiments, the biological filtration barrier comprises podocytes (also termed visceral epithelial cells). More specifically, the biological filtration barrier is a podocyte (filtration) barrier preventing blood plasma proteins from entering the second (urine) circuit. The podocyte filtration barrier preferably has filtration slits setting an upper limit of about 69 kDa for serum proteins to enter the second (urine) circuit. In various embodiments, the podocyte filtration barrier has a pore size setting a limit for albumin, preferably serum albumin (or blood albumin), to enter the second (urine) circuit.

In renal physiology, reabsorption (or tubular reabsorption) is the process by which the nephron removes water and solutes from the tubular fluid (pre-urine) and returns them to the circulating blood. The glomerular filtrate becomes more concentrated, which is one of the steps in forming urine. Reabsorption allows many useful solutes (primarily glucose and amino acids), salts and water to return to the blood circulation. As described herein, the reabsorption unit of the novel microfluidic device disclosed herein comprises a barrier that allows reabsorption of fluid from the second circuit (urine circuit) to the first circuit (blood circuit). In various embodiments of the present disclosure, the reabsorption barrier comprised by the reabsorption unit may be a (porous) mechanical barrier mimicking the reabsorption barrier. Preferably, the reabsorption barrier is a biological barrier, more preferably the biological reabsorption barrier comprises renal tubule cells (or renal tubular cells). More specifically, the biological reabsorption barrier is a renal tubular cell (reabsorption) barrier returning water, salt and solutes (the latter including proteins) into the first circuit (blood circuit). As described herein, reabsorption may be considered as the flow of glomerular filtrate from the reabsorption barrier (specifically from the tubular cells) into the first circuit (blood circuit), thereby allowing the selective passage of certain substances, in particular water, salt and solutes, back into the first circuit (blood circuit). In various embodiments, the renal tubular cell may be considered as renal proximal tubular cell.

The second circuit of the novel microfluidic device disclosed herein may comprise a compartment, which does not serve as a cell culture compartment, but which serves as a (medium) reservoir compartment. Preferably, such a (medium) reservoir compartment may exclusively serve as a "sampling reservoir" or "sampling chamber/compartment" for taking samples from the second circuit, and/or which exclusively serves as a "sample reservoir" or "sample chamber/compartment" for adding a test sample to the second circuit of the microfluidic. Accordingly, in preferred embodiments, the second circuit comprises no cell culture compartments other than the two compartments/units making up the filtration unit and the reabsorption unit, while including a separate (further) reservoir compartment, which serves to take samples (sampling compartment) from the second circuit, and/or which serves as a "sample reservoir" or "sample chamber/compartment" for adding a test sample to the second circuit of the microfluidic system.

The reservoir compartment may be used for adding a nutrient solution to the system, which is why it is also called a medium reservoir compartment. Accordingly, the reservoir compartment may serve three functions, sampling and feeding and adding test samples, however, does not serve as a cell culture compartment carrying an organ equivalent.

The first circuit of the novel microfluidic device disclosed herein comprises a compartment, which does not serve as a cell culture compartment, but which serves as a (medium) reservoir compartment. Preferably, such a (medium) reservoir compartment may exclusively serve to take samples from the first circuit. Also, such a reservoir compartment may be used for adding a nutrient solution to the system, which is why it may be called a medium reservoir compartment. Accordingly, the reservoir compartment of the first circuit may serve both functions, sampling and feeding, however, does not serve as a cell culture compartment carrying an organ equivalent. Accordingly, in preferred embodiments, the first circuit may comprise a reservoir compartment in addition to the one or more cell culture compartments, which serves to take samples (sampling compartment) from the first circuit and/or to add medium (nutrient solution) to the system.

In preferred embodiments of the present disclosure, at least one of the cell culture compartments of the first circuit of the novel microfluidic device comprises a tissue mimicking liver function ("liver equivalent").

In other preferred embodiments of the present disclosure, at least one of the cell culture compartments of the first circuit of the novel microfluidic device comprises a liver equivalent, and at least one of the cell culture compartments of the first circuit comprises an intestine equivalent. Such an embodiment is particularly useful to accomplish ADME profiling because the microfluidic chip then comprises organ equivalents of intestine and liver (first circuit), and kidney (second circuit). As described herein, the organ equivalents of intestine, liver, and kidney may be considered as "ADME" organ equivalents. In various embodiments, the novel microfluidic device may comprise one or more additional "non-ADME" organ equivalents, which are different from the "ADME" organ equivalents of intestine, liver, and kidney. In various embodiments, such additional "non-ADME" organ equivalents include, but are not limited thereto, a neuronal equivalent, a skin equivalent, or a respiratory tract equivalent (preferably lung equivalent).

In still other preferred embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a neuronal equivalent, thereby establishing an ADME-N chip.

In other preferred embodiments of the present disclosure, at least one of the cell culture compartments of the first circuit of the novel microfluidic device comprises a liver equivalent, and at least one of the cell culture compartments of the first circuit comprises a skin equivalent. Such an embodiment is also useful to accomplish ADME profiling because the microfluidic chip then comprises organ equivalents of skin and liver (first circuit), and kidney (second circuit). The administration of a test sample (test drug) is then performed via the skin equivalent. Such embodiments are particularly useful for testing cosmetics and/or consumer products, including creams.

In other preferred embodiments of the present disclosure, at least one of the cell culture compartments of the first circuit of the novel microfluidic device comprises a liver equivalent, and at least one of the cell culture compartments of the first circuit comprises a respiratory tract equivalent, specifically a lung equivalent. Such an embodiment is also useful to accomplish ADME profiling because the microfluidic chip then comprises organ equivalents of respiratory tract/lung and liver (first circuit), and kidney (second circuit). The administration of a test sample (test drug) is then performed via the respiratory tract/lung equivalent. Such embodiments are particularly useful for testing substances, including drugs, intended for application via the respiratory tract/lung.

In various other embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a skin equivalent.

In various further embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a respiratory tract, preferably lung, equivalent.

In still other embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a skin equivalent, and at least one of the cell culture compartments of the first circuit comprises a respiratory tract, preferably lung, equivalent.

In still further embodiments of the present disclosure, at least one of the cell culture compartments of the first circuit of the novel microfluidic device comprises a liver equivalent, and at least one of the cell culture compartments of the first circuit comprises an intestine equivalent, and at least one of the cell culture compartments of the first circuit comprises a skin equivalent, and at least one of the cell culture compartments of the first circuit comprises a respiratory tract, preferably lung, equivalent, and at least one of the cell culture compartments of the first circuit comprises a neuronal equivalent.

In various embodiments of the present disclosure, at least one of the cell culture compartments of the first circuit of the novel microfluidic device comprises a liver equivalent, and at least one of the cell culture compartments of the first circuit comprises an intestine equivalent, at least one of the cell culture compartments of the first circuit comprises a neuronal equivalent, and at least one of the cell culture compartments of the first circuit comprises a skin equivalent.

In various embodiments of the present disclosure, at least one of the cell culture compartments of the first circuit of the novel microfluidic device comprises a liver equivalent, and at least one of the cell culture compartments of the first circuit comprises an intestine equivalent, at least one of the cell culture compartments of the first circuit comprises a neuronal equivalent, and at least one of the cell culture compartments of the first circuit comprises a respiratory tract, preferably lung, equivalent.

Preferred organ equivalents include kidney equivalent, liver equivalent, intestine equivalent, preferably small intestine equivalent, neuronal (brain) equivalent, respiratory tract/lung equivalent, and skin equivalent. In case of kidney (or renal) equivalent, the equivalent specifically means an equivalent of the glomerulus and tubulus. More specifically, such equivalents of glomerulus and tubulus are arranged in separate units (filtration unit and reabsorption unit, respectively) of the second circuit of the novel microfluidic device provided herein.

As described herein, a "cell culture compartment" comprises or contains an organ equivalent, which encompasses cells and tissues mimicking organ function. Thus, in various embodiments, the terms "organ equivalent" and "cells/tissue mimicking organ function" may be used interchangeably herein.
In various embodiments, an organ equivalent or a tissue mimicking an organ function is a primary organ tissue, e.g., a primary liver tissue or a primary intestine tissue. Preferably, the primary organ tissue comprises epithelial cells of the respective organ, *i.e.,* epithelial cells of, *e.g.,* the gastrointestinal tract, the skin, or the respiratory tract.
In various embodiments, a tissue mimicking an organ function may be a tissue comprising epithelial cells of the respective organ, *i.e.,* epithelial cells of, *e.g.,* the gastrointestinal tract, the skin, or the respiratory tract. In various other embodiments, a tissue mimicking an organ function may be a tissue comprising cells of a cell line, including cells obtainable from a commercially available cell line.
In various other embodiments, a tissue mimicking organ function is a tissue comprising cells derived from induced pluripotent stem cells (iPSCs). Specifically, the cells may be derived (*i.e.,* differentiated) from a single (*i.e.,* one and the same) donor iPSC, which in turn has been obtained (*i.e.,* reprogrammed) from a single type of somatic cell. For example, the cells may be derived (*i.e.,* differentiated) from a single (i.e., one and the same) iPSC, which has been obtained (*i.e.,* reprogrammed) from, *e.g.,* a somatic skin cell, white blood cell, renal cell, adipocytes, or the like.

As described elsewhere herein, cells or tissue mimicking organ function include organoids and spheroids. Thus, in various embodiments, an organ equivalent specifically means an organoid or spheroid of a certain organ functionality, *e.g.,* an organoid of intestinal cells or hepatocytes, or a spheroid of intestinal cells or hepatocytes.

The cells, tissues and/or organs of the microfluidic device provided by the present disclosure can be modeled at a two-dimensional (2D), three-dimensional (3D), or organotypic complexity. 2D means suspension of monolayer cell cultures, while 3D includes, *e.g.,* multilayer cultures of different geometry, including, *e.g.,* organoids and spheroids. Organotypic differs from 3D by capturing further features of the *in vivo* organ architecture.

In various embodiments, a cell culture compartment comprising an organ equivalent is a cell culture compartment comprising an organoid. As described herein, the term "organoid" may be considered to mean or represent the smallest functional organ or tissue unit. A selection of organoid histologies, all with relevant functionality and highly variable conglomerate geometry, has been published for 15 key human organs by Marx et al. (Marx et al. 2012, Altern Lab Anim 40, 235-257). As further described herein, an organoid according to the present disclosure specifically means an organoid of iPSC-derived cells, however without being limited thereto. As further described herein, an organoid may be considered as a cell/tissue culture, which is mimicking organ function. As further described herein, an organoid may be typically characterized by any of the following characteristics, without being limited thereto: (i) (at least) three-dimensional multicellular construct or structure; (ii) collection of organ-specific cell types; (iii) if generated from stem cells (preferably iPSCs), it is usually recapitulating developmental organogenesis program; (iv) self-organization (ability of the cells to self-assemble or/and self-organize into tissues).

In various embodiments, the organoids are embedded into an extracellular matrix, preferably into an extracellular gel matrix, more preferably into a basement membrane-like extracellular matrix extract (typically resembling tissue extracellular environment). In various embodiments, the basement membrane-like extracellular (gel) matrix extract comprises a gelatinous protein mixture. Preferably, the gelatinous protein mixture is gelatinous protein mixture secreted by Engelbreth-Holm-Swarm (EHS) mouse sarcoma cells. More preferably, the basement membrane-like extracellular (gel) matrix is the Matrigel® basement membrane matrix (Corning). In various embodiments of the present disclosure, the extracellular (gel) matrix is a hydrogel. In various embodiments of the present disclosure, the extracellular (gel) matrix is a collagen, agarose, aginate or Matrigel® hydrogel. In various further embodiments, the hydrogel is a synthetic hydrogel.

In various embodiments, a cell culture compartment comprising an organ equivalent is a cell culture compartment comprising spheroids mimicking organ function. As described herein, a spheroid according to the present disclosure specifically means a spheroid of iPSC-derived cells, however without being limited thereto. As further described herein, a spheroid may be considered as an aggregation or self-assembly of (spherical clusters of) cells, closely resembling *in vivo* organ tissues. A spheroid has, like an organoid, a three-dimensional structural architecture. As compared to organoids, it is considered that there is a higher order of self-assembly in organoids as opposed to spheroid cultures. In various embodiments, an organoid may be considered as a cell/tissue culture mimicking organ function.

Preferred organoids include kidney organoid, liver organoid, intestine organoid, preferably small intestine organoid, neuronal (brain) organoid, respiratory tract/lung organoid, and skin organoid. In case of a kidney (or renal) organoid, the organoid specifically means an organoid of the glomerulus and tubulus. More specifically, such organoids of glomerulus and tubulus are arranged in separate units (filtration unit and reabsorption unit, respectively) of the second circuit of the novel microfluidic device provided herein.

In various embodiments, the filtration unit and the reabsorption unit of the second circuit may be considered as kidney or renal equivalent. Preferably, the kidney or renal equivalent means a kidney or renal organoid. As described herein, a kidney or renal organoid preferably means organoids of podocytes and renal tubular cells, more preferably organoids of iPSC-derived podocytes and iPSC-derived renal tubular cells. Likewise, a liver organoid preferably means an organoid of hepatocytes, more preferably organoids of iPSC-derived hepatocytes. Likewise, a (small) intestine organoid preferably means an organoid of (small) intestinal cells, more preferably an organoid of iPSC-derived (small) intestinal cells. Likewise, a neuronal organoid preferably means an organoid of neuronal cells, more preferably an organoid of iPSC-derived neuronal cells. Likewise, respiratory tract (preferably lung) organoid preferably means an organoid of cells of the respiratory tract, preferably lung cells, more preferably lung epithelial cells. Preferably, the cells of the respiratory tract or the lung cells are iPSC-derived cells of the respiratory tract or iPSC-derived lung cells, respectively.

In various embodiments of the present disclosure, the novel microfluidic device has an arrangement of cell culture compartments in accordance with the layout shown in Fig. 1, however, without being limited thereto. Accordingly, in various preferred embodiments, the cell culture compartments comprising the intestine equivalent and the liver equivalent are arranged such that the latter ("liver equivalent") is located downstream of the former ("intestine equivalent") (in accordance with the layout for the "blood circuit" depicted in Fig. 1, however, without being limited thereto). Preferably, the cell culture compartments comprising the "liver equivalent" and the "intestine equivalent" are arranged in a separate microchannel, which is a branch of the main microchannel structure (in accordance with the layout for the "blood circuit" depicted in Fig. 1, however, without being limited thereto). Preferably, no other cell culture compartments are located between the cell culture compartments of the intestine equivalent and the liver equivalent. In various embodiments, the first and second circuit may comprise a branched microfluidic network (or branched network of microfluidic channels). In further preferred embodiments, the novel microfluidic device has an arrangement of cell culture compartments such that a separate cell culture compartment, in addition to the cell culture compartments comprising the "liver equivalent" and the "intestine equivalent" and comprising a further organ equivalent different from the "liver equivalent" and the "intestine equivalent", is arranged in parallel to the cell culture compartments comprising the "liver equivalent" and the "intestine equivalent". More preferably, such an additional cell culture compartment is arranged in a separate microchannel branch arranged in parallel to the microchannel branch comprising the cell culture compartments comprising the "liver equivalent" and the "intestine equivalent" (in accordance with the layout for the "blood circuit" depicted in Fig. 1, however, without being limited thereto). Preferably, the additional cell culture compartment comprising a further organ equivalent different from the "liver equivalent" and the "intestine equivalent" comprises a neuronal equivalent.
In case the microfluidic device contains a separate medium reservoir or sampling reservoir, this is preferably arranged in the main microchannel structure, *i.e.,* is not arranged in a branch microchannel structure (in accordance with the layout for the "blood circuit" depicted in Fig. 1, however, without being limited thereto).
Furthermore, in case the microfluidic device contains a micropump, the micropump is preferably arranged in the main microchannel structure, *i.e.,* is not arranged in a branch microchannel structure (in accordance with the layout for the "blood circuit" depicted in Fig. 1, however, without being limited thereto).
In various preferred embodiments, the second circuit does not show a branched network, but rather is an unbranched circuit or circulation system. The terms "circuit" and "circulation system" may be used interchangeably herein.

The novel microphysiological system preferably is a closed channel system. Accordingly, the first and second circuit of the novel microfluidic device form a closed circuit, more specifically a closed circuit of microfluidic channels. More specifically, the novel microphysiological system may be considered as a self-supported or self-contained microphysiological system. The closed channel system including the two renal functional units (filtration unit and reabsorption unit) enable specifically assaying kidney toxicity of test drugs. In preferred embodiments, the microphysiological system or microfluidic device is operated with a blood-mimicking liquid solution and/or a urine mimicking liquid solution in the second circuit.

In various aspects of the present disclosure, at least one of the cell culture compartments of the first circuit of the novel microfluidic device comprises an iPSC-derived liver equivalent, and at least one of the cell culture compartments of the first circuit comprises an iPSC-derived intestine equivalent, and at least one of the cell culture compartments of the first circuit comprises an iPSC-derived neuronal equivalent, and the second circuit of the novel microfluidic device comprises renal proximal tubule epithelial cells (RPTEC), more specifically RPTECs seeded in the reabsorption unit. Preferably, the at least one cell culture compartment comprising an iPSC-derived intestine equivalent comprises organoids of iPSC-derived intestinal cells. More preferably, the organoids of iPSC-derived intestinal cells are embedded into an extracellular matrix, preferably into an extracellular gel matrix, more preferably into a basement membrane-like extracellular (gel) matrix extract (typically resembling tissue extracellular environment). In various embodiments, the basement membrane-like extracellular (gel) matrix extract comprises a gelatinous protein mixture. Preferably, the basement membrane-like extracellular matrix is the Matrigel® basement membrane matrix (Corning).
In further preferred embodiments, the at least one cell culture compartment comprising an iPSC-derived intestine equivalent, specifically organoids of iPSC-derived intestinal cells, furthermore comprises fibroblasts and endothelial cells, more preferably iPSC-derived fibroblasts and iPSC-derived endothelial cells, or iPSC-derived fibroblasts and primary microvascular endothelial cells. The primary microvascular endothelial cells or iPSC-derived endothelial cells are preferably seeded on the basolateral side of the cell culture compartment.
Furthermore, the at least one cell culture compartment comprising an iPSC-derived neuronal equivalent preferably comprises iPSC-derived neuronal spheroids. More preferably, the at least one cell culture compartment comprising an iPSC-derived neuronal equivalent, specifically iPSC-derived neuronal spheroids, comprises a bioprinted blood brain barrier. More specifically, the at least one cell culture compartment comprising an iPSC-derived neuronal equivalent, specifically iPSC-derived neuronal spheroids, is enriched with endothelial cells, preferably iPSC-derived endothelial cells, bioprinted under the neuronal spheroids.
Furthermore, the at least one cell culture compartment comprising an iPSC-derived liver equivalent preferably comprises spheroids of iPSC-derived hepatocytes. More preferably, the at least one cell culture compartment comprising an iPSC-derived liver equivalent, specifically spheroids of iPSC-derived hepatocytes, further comprises fibroblasts, preferably iPSC-derived fibroblasts. In preferred embodiments, the iPSC-derived liver equivalent is accomplished by placing spheroids of iPSC-derived hepatocytes with iPSC-derived fibroblasts into the respective cell culture compartment.
In various embodiments, the RPTECs comprise a renal proximal tubule epithelial cell line.

In various aspects of the present disclosure, at least one of the cell culture compartments of the first circuit of the novel microfluidic device comprises primary liver tissue, and at least one of the cell culture compartments of the first circuit comprises a primary intestine tissue, preferably a primary small intestine tissue, and at least one of the cell culture compartments of the first circuit comprises an iPSC-derived neuronal equivalent, and the second circuit of the novel microfluidic device comprises renal proximal tubule epithelial cells (RPTECs), more specifically RPTECs seeded in the reabsorption unit. In various embodiments, the primary liver tissue comprises primary (human) hepatocytes. The primary liver tissue may be a commercially available liver model, *e.g.,* the 3D InSight™ Human Liver Microtissues XL, InSphero. In various embodiments, the primary liver tissue or liver model may comprise 50 spheroids, each containing 3,000 primary hepatocytes. Furthermore, in preferred embodiments, the primary intestine tissue comprises primary (human) cell-derived intestine epithelial and endothelial cells, and more preferably further comprises (human) fibroblasts. The primary intestine tissue may be a commercially available intestine model, *e.g.,* the 3D Epilntestinal® tissue model (MatTek Corporation). Furthermore, the at least one cell culture compartment comprising an iPSC-derived neuronal equivalent preferably comprises iPSC-derived neuronal spheroids. More preferably, the at least one cell culture compartment comprising an iPSC-derived neuronal equivalent, specifically iPSC-derived neuronal spheroids, comprises a bioprinted blood brain barrier. More specifically, the at least one cell culture compartment comprising an iPSC-derived neuronal equivalent, specifically iPSC-derived neuronal spheroids, is enriched with endothelial cells, preferably iPSC-derived endothelial cells, that are bioprinted under the neuronal spheroids.
In various embodiments, the RPTECs comprise a renal proximal tubule epithelial cell line.

In various aspects of the present disclosure, at least one of the cell culture compartments of the first circuit of the novel microfluidic device comprises an iPSC-derived liver equivalent, and at least one of the cell culture compartments of the first circuit comprises an iPSC-derived intestine equivalent, and at least one of the cell culture compartments of the first circuit comprises an iPSC-derived neuronal equivalent, and the second circuit of the novel microfluidic device comprises an iPSC-derived renal equivalent. Preferably, the at least one cell culture compartment comprising an iPSC-derived intestine equivalent comprises organoids of iPSC-derived intestinal cells. More preferably, the organoids of iPSC-derived intestinal cells are embedded into an extracellular matrix, preferably into an extracellular gel matrix, more preferably into a basement membrane-like extracellular (gel) matrix extract (typically resembling tissue extracellular environment). In various embodiments, the basement membrane-like extracellular (gel) matrix extract comprises a gelatinous protein mixture. Preferably, the basement membrane-like extracellular matrix is the Matrigel® basement membrane matrix (Corning).
In further preferred embodiments, the at least one cell culture compartment comprising an iPSC-derived intestine equivalent, specifically organoids of iPSC-derived intestinal cells, furthermore comprises fibroblasts and endothelial cells, more preferably iPSC-derived fibroblasts and iPSC-derived endothelial cells, or iPSC-derived fibroblasts and primary microvascular endothelial cells. The primary microvascular endothelial cells or iPSC-derived endothelial cells are preferably seeded on the basolateral side of the cell culture compartment.
Furthermore, the at least one cell culture compartment comprising an iPSC-derived neuronal equivalent preferably comprises iPSC-derived neuronal spheroids. More preferably, the at least one cell culture compartment comprising an iPSC-derived neuronal equivalent, specifically iPSC-derived neuronal spheroids, comprises a bioprinted blood brain barrier. More specifically, the at least one cell culture compartment comprising an iPSC-derived neuronal equivalent, specifically iPSC-derived neuronal spheroids, is enriched with endothelial cells, preferably iPSC-derived endothelial cells, seeded on the basolateral side of the cell culture compartment.
Furthermore, the at least one cell culture compartment comprising an iPSC-derived liver equivalent preferably comprises spheroids of iPSC-derived hepatocytes. More preferably, the at least one cell culture compartment comprising an iPSC-derived liver equivalent, specifically spheroids of iPSC-derived hepatocytes, further comprises fibroblasts, preferably iPSC-derived fibroblasts. In preferred embodiments, the iPSC-derived liver equivalent is accomplished by placing spheroids of iPSC-derived hepatocytes with iPSC-derived fibroblasts into the respective cell culture compartment.

In various aspects of the present disclosure, at least one of the cell culture compartments of the first circuit of the novel microfluidic device comprises primary liver tissue, and at least one of the cell culture compartments of the first circuit comprises a primary intestine tissue, preferably a primary small intestine tissue, and at least one of the cell culture compartments of the first circuit comprises a primary neuronal tissue, and the second circuit of the novel microfluidic device comprises a primary renal tissue, preferably a primary renal tissue in the reabsorption unit, more preferably a primary tubular (epithelial cell) tissue in the reabsorption unit, and a primary glomerular tissue in the filtration unit. Preferably, the primary renal tissue comprises renal epithelial cells, more specifically renal proximal tubule epithelial cells (RPTECs), wherein the renal epithelial cells or RPTECs may be seeded in the reabsorption unit, and the primary glomerular tissue comprises podocytes, which may be seeded in the filtration unit. In various embodiments, the primary liver tissue comprises primary (human) hepatocytes. The primary liver tissue may be a commercially available liver model, e.g., the 3D InSight™ Human Liver Microtissues XL, InSphero. Furthermore, in preferred embodiments, the primary intestine tissue comprises primary (human) cell-derived intestine epithelial and endothelial cells, and more preferably further comprises (human) fibroblasts. The primary intestine tissue may be a commercially available intestine model, e.g., the 3D Epilntestinal® tissue model (MatTek Corporation).
Furthermore, the primary neuronal tissue preferably comprises primary neuronal cells. In still further preferred embodiments, the least one cell culture compartment comprising the primary neuronal tissue, specifically primary neuronal cells, further comprises a bioprinted blood brain barrier. More specifically, the at least one cell culture compartment comprising the primary neuronal tissue, specifically primary neuronal cells, is enriched with endothelial cells, that may be either seeded on the basolateral side of the cell culture compartment, or are bioprinted under the neuronal spheroids.

In various aspects of the present disclosure, at least one of the cell culture compartments of the first circuit of the novel microfluidic device comprises a liver organoid, and at least one of the cell culture compartments of the first circuit comprises an intestine organoid, preferably a small intestine organoid, and the second circuit of the novel microfluidic device comprises a renal organoid in the reabsorption unit and/or the filtration unit. In various embodiments, at least one additional cell culture compartment of the first circuit further comprises an additional organoid, which is different from the liver organoid, the intestine organoid (including the small intestine organoid), and the renal organoid. Such an additional organoid may be considered as "non-ADME" organoid, while the liver organoid, the intestine organoid (including the small intestine organoid), and the renal organoid may be considered as "ADME" organoids. In various embodiments, such an additional (non-ADME) organoid may be any of a neuronal organoid, a skin organoid, or a respiratory tract (preferably lung) organoid. In various embodiments, at least two additional cell culture compartments of the first circuit further comprise two additional "non-ADME" organoids (one organoid per additional cell culture compartment), which are different from the liver organoid, the intestine organoid (including the small intestine organoid), and the renal organoid. In various embodiments, the two additional (non-ADME) organoids may be selected from any of a neuronal organoid, a skin organoid, and/or a respiratory tract (preferably lung) organoid. In still other embodiments, at least three additional cell culture compartments of the first circuit further comprise three additional "non-ADME" organoids (one organoid per additional cell culture compartment). In various embodiments, the three additional "non-ADME" organoids are a neuronal organoid, a skin organoid, and/or a respiratory tract (preferably lung) organoid. Preferably, the organoids are organoids of iPSC-derived organ cells. Specifically, the liver organoid preferably is an organoid of iPSC-derived hepatocytes, and the intestine organoid preferably is an organoid of iPSC-derived intestinal cells, more preferably an organoid of iPSC-derived cells of the small intestine, and the neuronal organoid preferably is an organoid of iPSC-derived neuronal cells, and the renal organoid preferably is an organoid of iPSC-derived renal cells.

In various aspects of the present disclosure, at least one of the cell culture compartments of the first circuit of the novel microfluidic device comprises a liver spheroid, and at least one of the cell culture compartments of the first circuit comprises an intestine spheroid, preferably a small intestine organoid, and the second circuit of the novel microfluidic device comprises a renal spheroid in the reabsorption unit and/or the filtration unit. In various embodiments, at least one additional culture compartment of the first circuit further comprises an additional spheroid which is different from the liver spheroid, the intestine spheroid (including the small intestine spheroid), and the renal spheroid. Such an additional spheroid may be considered as "non-ADME" spheroid, while the liver spheroid, the intestine spheroid (including the small intestine spheroid), and the renal spheroid may be considered as "ADME" spheroids. In various embodiments, such an additional (non-ADME) spheroid may be any of a neuronal spheroid, a skin spheroid, and/or a respiratory tract (preferably lung) spheroid.In various embodiments, at least two additional cell culture compartments of the first circuit further comprise two additional "non-ADME" spheroids (one spheroid per additional cell culture compartment), which are different from the liver spheroid, the intestine spheroid (including the small intestine spheroid), and the renal spheroid. In various embodiments, the two additional (non-ADME) spheroids may be selected from any of a neuronal spheroid, a skin spheroid, and/or a respiratory tract (preferably lung) spheroid.In still other embodiments, at least three additional cell culture compartments of the first circuit further comprise three additional "non-ADME" spheroids (one spheroid per additional cell culture compartment). In various embodiments, the three additional "non-ADME" spheroids are a neuronal spheroid, a skin spheroid, and/or a respiratory tract (preferably lung) spheroid. Preferably, the spheroids are spheroids of iPSC-derived organ cells. Specifically, the liver spheroid preferably is a spheroid of iPSC-derived hepatocytes, and the intestine spheroid preferably is a spheroid of iPSC-derived intestinal cells, more preferably a spheroid of iPSC-derived cells of the small intestine, and the neuronal spheroid preferably is a spheroid of iPSC-derived neuronal cells, and the renal spheroid preferably is a spheroid of iPSC-derived renal cells.

In various embodiments, a cell culture compartment may comprise one or more organ equivalents, *e.g.,* a neuronal equivalent along or in combination with an intestinal equivalent. In various other embodiments, each cell culture compartment (or a single cell culture compartment) may comprise only one organ equivalent, following the principle one individual organ equivalent per cell culture compartment. On the other hand, if, *for example,* at least one of the cell culture compartments of the first circuit of the novel microfluidic device comprises a liver equivalent, this means that there might be two cell culture compartments of the first circuit each comprising a liver equivalent. However, in preferred embodiments, the novel microfluidic device comprises each organ equivalent only once, following the principle one individual organ equivalent per microfluidic device (or per microphysiological system).

The present disclosure provides the use of a novel microfluidic device described herein in analytical testing, diagnostics, research, target validation, toxicity studies, tissue engineering, tissue manufacturing, drug screening, and/or pharmacokinetic-pharmacodynamic analysis.

The present disclosure enables operating a microphysiological system and establishes the so-called ADME profiling based on the novel microfluidic device provided herein. Accordingly, the present disclosure provides a method of operating a microphysiological system making use of the novel microfluidic devices described herein, and further provides an ADME (absorption, distribution, metabolism, excretion) assay comprising the novel microfluidic devices provided by the present disclosure. Further disclosed herein is an ADMET (absorption, distribution, metabolism, excretion, toxicity) assay comprising the novel microfluidic devices described herein.
Specifically in the drug discovery process, *in vitro* ADME profiling (or screening) provides a basis for selecting new molecular entities and lead compounds that have desirable drug metabolism, pharmacokinetics, or safety profiles, which are necessary for drug candidate selection and late-stage pre-clinical and clinical development. The ADME(T) profile (or properties) of a drug (test substance) allow the drug developer to understand the safety and efficacy data required for regulatory approval.
In the method of operating a microphysiological system, the culture time may last weeks or even months, enabling disease modeling and repeated dose substance exposure.

The present disclosure provides a method of performing an ADME (absorption, distribution, metabolism, excretion) profiling making use of the novel microfluidic device provided by the present disclosure. The present disclosure also provides a method of performing an ADMET (absorption, distribution, metabolism, excretion, toxicity) profiling making use of the novel microfluidic device described herein. Further disclosed herein is a method of generating an ADME profile, or an ADMET profile of a (test) drug comprising performing an ADME screening or ADMET screening, respectively, using the novel microfluidic device provided by the present disclosure.

The method of operating a microphysiological system as disclosed herein preferably comprises selectively adding a nutrient solution to the microphysiological system via the cell culture compartment comprising the gastrointestinal tract equivalent, preferably the intestine equivalent, more preferably the small intestine equivalent. The organ equivalent preferably comprises tissue comprising epithelial cells of the gastrointestinal tract, preferably of the intestine, even more preferably of the small intestine. In order to mirror physiological *in vivo* conditions, the present disclosure is preferably operating the microphysiological system with selectively adding the nutrient solution to the microphysiological system via the cell culture compartment comprising the gastrointestinal tract equivalent, preferably the intestine equivalent, more preferably the small intestine equivalent.

In various embodiments, a nutrient solution is selectively added to the microphysiological system via the cell culture compartment comprising the skin equivalent, and/or respiratory tract equivalent, more specifically lung equivalent.

As described herein, a nutrient solution specifically comprises substances (nutrients) which are essential for cells or a body to grow or to recover (including amino acids, salts (electrolytes), and glucose), and calories in the form of carbohydrates. Specifically, a nutrient solution as described herein is a nutrient solution for cell proliferation. As described herein, the terms "nutrient solution" and "cell culture medium" may be used interchangeably herein.

It has surprisingly been found that the novel microfluidic chips provided herein can establish homeostasis in the microphysiological system. This was shown by constant levels of glucose and LDH (Fig. 3). A constant glucose concentration suggests constant consumption and an active regulation of the available glucose. The glucose levels have reached equal levels in all compartments. It has furthermore surprisingly been found that feeding selectively through the intestine equivalent (apically through the intestine equivalent) was sufficient to supply both, the "blood" circuit and the "urine" circuit. Also, LDH activity has been shown rather constant throughout the experiments. As compared to glucose, LDH levels are different in each compartment. The barrier function of the intestine equivalent and the renal equivalent is shown by different concentrations of LDH in all three "compartments" *(i.e.,* blood circuit -> medium reservoir 1; urine circuit -> medium reservoir 2; and intestine compartment). The barriers, thus, differentiate between the crossing substances. The active or passive penetration of glucose, while retaining other substances, indicate practical interactions between subsets of the chip system.

Accordingly, the present disclosure provides a method of mimicking homeostasis comprising operating a microphysiological system comprising a first circuit with one or more cell culture compartments and a second circuit comprising a filtration unit and a reabsorption unit, wherein both circuits are connected with each other via the filtration unit and the reabsorption unit. In accordance with the (preferred) embodiments described elsewhere herein with respect to the novel microfluidic device, the filtration unit comprised by the second circuit of the microphysiological system preferably comprises a filtration barrier that selectively allows the passage of molecules from the first circuit to the second circuit based on size and charge of the molecules. More preferably, the filtration barrier is a biological barrier, even more preferably wherein the biological barrier comprises podocytes.

Also in accordance with the (preferred) embodiments described elsewhere herein with respect to the novel microfluidic device, the reabsorption unit comprised by the second circuit of the microphysiological system preferably comprises a barrier that allows reabsorption of fluid from the second circuit to the first circuit. More preferably, the reabsorption barrier is a biological barrier, even more preferably wherein the biological barrier comprises renal tubule cells.

In various embodiments of the novel method of mimicking homeostasis, a nutrient solution is selectively added to the microphysiological system via the cell culture compartment (of the first circuit) comprising the gastrointestinal tract equivalent, more specifically the intestine equivalent, even more specifically the small intestine equivalent. In various embodiments, the organ equivalent comprises epithelial cells, or tissue comprising epithelial cells, of the gastrointestinal tract, preferably of the intestine, even more preferably of the small intestine.

The (preferred) embodiments described herein in relation to the novel microfluidic device likewise apply to the novel method of mimicking homeostasis comprising operating a microphysiological system as described herein. Thus, (preferred) embodiments described elsewhere herein in relation to the novel microfluidic device are also preferred embodiments in relation to the novel method of mimicking homeostasis comprising operating a microphysiological system. More specifically, the (preferred) embodiments described elsewhere herein in relation to the novel microfluidic device are also preferred embodiments of the microphysiological system comprised by the novel method of mimicking homeostasis.

The present disclosure further provides a method of detecting an analyte in a microphysiological system making use of a novel microfluidic device described herein, wherein the method comprises adding a test sample to a cell culture compartment of the first circuit, preferably wherein the said cell culture compartment comprises an organ equivalent selected from any of gastrointestinal tract equivalent, more specifically intestine equivalent, skin equivalent, and/or respiratory tract equivalent, more specifically lung equivalent. The organ equivalent preferably comprises tissue comprising epithelial cells of the gastrointestinal tract, the skin, or the respiratory tract. In preferred embodiments, a test sample is added to a cell culture compartment comprising the gastrointestinal tract equivalent, more specifically the intestine equivalent. More preferably, the test sample is selectively added to cell culture compartment comprising the gastrointestinal tract equivalent, specifically the intestine equivalent, more specifically the small intestine equivalent.
In various embodiments, a test sample is selectively added to the microphysiological system via the cell culture compartment comprising the skin equivalent, and/or respiratory tract equivalent, more specifically lung equivalent.
In various other embodiments, a test sample is selectively added to the microphysiological system via a cavity different from the cell culture compartments comprising the organ equivalents. Such a cavity may be the "medium reservoir" of the blood circuit and/or the urine circuit. As described elsewhere herein, such a "medium reservoir" is intended to be a "sampling reservoir" or "sampling chamber/compartment" which exclusively serves to take a sample or culture supernatant from the microfluidic system, and/or which is intended to be a "sample reservoir" or "sample chamber/compartment" which exclusively serves to add a test sample to the microfluidic system.
In various embodiments, the test sample is a liquid test sample, which is preferably added to the microphysiological system via the cell culture compartment comprising the gastrointestinal tract equivalent, specifically the intestine equivalent, more specifically the small intestine equivalent. In various embodiments, the test sample is a cosmetic or consumer product, including a cream, which is preferably added to the microphysiological system via the cell culture compartment comprising the skin equivalent.
The method of detecting an analyte according to the present disclosure may comprise taking a sample from a "medium reservoir" of the blood circuit and/or the urine circuit. As described elsewhere herein, such a "medium reservoir" may be intended to be a "sampling reservoir" or "sampling chamber/compartment" which serves to take a sample or culture supernatant from the microfluidic system, and/or which may be intended to be a "sample reservoir" or "sample chamber/compartment" which serves to add a test sample to the microfluidic system, but which does not serve as a cell culture compartment for an organ equivalent.
The method of detecting an analyte according to the present disclosure may further comprise analyzing the sample taken from the microfluidic system via a sampling chamber for the presence of metabolites, preferably toxic metabolites. The method of detecting an analyte according to the present disclosure specifically allows detecting metabolites of drugs added as test samples to the microphysiological system.
The method of detecting an analyte according to the present disclosure may further comprise detecting an analyte in the fluid flow of the first circuit and/or the second circuit.
In preferred embodiments of the method of detecting an analyte, a test sample is added to or deposited in a cell culture compartment of the first circuit, which is the same or different from the cell culture compartment to which a nutrient solution is added.

Further disclosed herein is a method of culturing and/or maintaining cells comprising seeding a novel microfluidic device described herein with cells.

Still further disclosed herein is a kit for operating a microphysiological system comprising a novel microfluidic device described, and instructions for use.

The microfluidic device provided by the present disclosure may be in form of a plate or a chip. Plates are usually based on the microtiter plate dimensions and use passive, gravity-based microfluidic flow or active on-board pumping. Chips of microscope slide size and other formats may be operated by an active or passive microfluidic flow, thereby emulating the shear stress at physiological intra-capillary or interstitial rates. In case of an active microfluidic flow, external or on-chip micropumps may be used. Preferably, the microfluidic device provided by the present disclosure is in form of a chip.

In various embodiments of the novel microfluidic device or the novel microphysiological system disclosed herein, the first circuit comprises one or more microfluidic channels that are lined with endothelial cells. As described elsewhere herein, the present disclosure demonstrates that shear stress promotes elongation of iPSC-derived endothelial cells in the channels of the multi-organ-chip (Fig. 11).

In various embodiments, the novel microfluidic device or the novel microphysiological system comprises a micropump, preferably a peristaltic micropump. In various embodiments, one of the two circuits (first and second circuit) may have a micropump (*i.e.,* "blood circuit micropump" or "urine circuit micropump"). In preferred embodiments, each of the two circuits (first and second circuit) has its own micropump (*i.e.,* "blood circuit micropump" and "urine circuit micropump"). The micropumps are preferably "on-chip" micropumps, in accordance with the preferred embodiment that the novel microfluidic device or the novel microphysiological system is a closed channel system.

As described herein, the novel methods provided herein may encompass a step of depositing and/or culturing an organ equivalent, preferably a tissue comprising (epithelial) cells or iPSC-derived (epithelial) cells of the respective organ, in a cell culture compartment of the first circuit.

In various embodiments, the intestine equivalent may (further) comprise fibroblasts, in particular iPSC-derived fibroblasts. Preferably, the intestine equivalent is an iPSC-derived intestine equivalent further comprising fibroblasts, in particular iPSC-derived fibroblasts. More preferably, the intestine equivalent is an iPSC-derived equivalent formed by a layer of an extracellular matrix, which is covered by fibroblasts, in particular iPSC-derived fibroblasts. The layer of an extracellular matrix preferably is a gel-based layer of an extracellular matrix. Even more preferably, the intestine equivalent is an organoid of iPSC-derived intestinal cells. Preferably, the intestine equivalent is an iPSC-derived equivalent, preferably an organoid of iPSC-derived intestinal cells, formed by a layer of a basement membrane-like extracellular matrix extract (typically resembling tissue extracellular environment), wherein the layer is covered by fibroblasts, in particular iPSC-derived fibroblasts. In various embodiments, the basement membrane-like extracellular matrix extract comprises a gelatinous protein mixture. Preferably, the basement membrane-like extracellular matrix is the Matrigel® basement membrane matrix (Corning).
In various embodiments, the intestine equivalent, which preferably is an organoid of iPSC-derived intestinal cells, is incorporated or embedded into the aforementioned extracellular matrix.

In various embodiments, the intestine equivalent, which preferably is an organoid of iPSC-derived intestinal cells, comprises a (closed) layer of endothelial cells, including primary microvascular or iPSC-derived endothelial cells (the latter are preferred), on the basolateral side of the cell culture compartment. In various embodiments, the intestine equivalent, which preferably is an organoid of iPSC-derived intestinal cells, comprises a (closed) layer of iPSC-derived endothelial cells on the basolateral side of the cell culture compartment.

In various embodiments, the intestine equivalent may (further) comprise fibroblasts and endothelial cells, in particular iPSC-derived fibroblasts and iPSC-derived endothelial cells, as described above. Accordingly, in various embodiments, the intestine equivalent is an iPSC-derived intestine equivalent formed by a layer of an extracellular matrix, which is covered by fibroblasts, in particular iPSC-derived fibroblasts, and wherein the intestine equivalent further comprises another layer of endothelial cells, preferably primary microvascular endothelial cells, on the basolateral side of the cell culture compartment. Alternatively, in various embodiments, the intestine equivalent is an iPSC-derived intestine equivalent formed by a layer of an extracellular matrix, which is covered by fibroblasts, in particular iPSC-derived fibroblasts, and wherein the intestine equivalent further comprises another layer of iPSC-derived endothelial cells on the basolateral side of the cell culture compartment.
In various embodiments, the intestine equivalent is incorporated or embedded into the aforementioned extracellular matrix.
Furthermore, as described above, the iPSC-derived intestine equivalent preferably is an organoid of iPSC-derived intestinal cells. Also, the layer of the extracellular matrix preferably is a gel-based layer of an extracellular matrix, more preferably a layer of a basement membrane-like extracellular matrix extract (typically resembling tissue extracellular environment). The basement membrane-like extracellular matrix extract may comprise a gelatinous protein mixture. Preferably, the basement membrane-like extracellular matrix is the Matrigel® basement membrane matrix (Corning).

In various embodiments, the neuronal equivalent may (further) comprise endothelial cells, in particular iPSC-derived endothelial cells. Preferably, the neuronal equivalent is an iPSC-derived equivalent further comprising endothelial cells, in particular iPSC-derived endothelial cells. More preferably, the endothelial cells, in particular iPSC-derived endothelial cells, are present on the basolateral side of the neuronal equivalent of the cell culture compartment comprising the neuronal equivalent. Alternatively, the neuronal equivalent is an iPSC-derived neuronal equivalent further comprising iPSC-derived endothelial cells bioprinted (and thus present) under the neuronal equivalent. Preferably, the iPSC-derived neuronal equivalent comprises spheroids or organoids of iPSC-derived neuronal cells, and the iPSC-derived endothelial cells are bioprinted (and thus present) under the neuronal spheroid or organoid of iPSC-derived neuronal cells. Preferably, the iPSC-derived neuronal equivalent comprises spheroids or organoids of iPSC-derived neuronal cells, which are incorporated or embedded into a layer of an extracellular matrix. Preferably, the iPSC-derived endothelial cells are bioprinted (and thus present) in the extracellular matrix under (basolateral) the neuronal spheroids or organoids of iPSC-derived neuronal cells. As described above, the extracellular matrix preferably is an extracellular gel matrix, more preferably a basement membrane-like extracellular matrix extract (typically resembling tissue extracellular environment). The basement membrane-like extracellular matrix extract may comprise a gelatinous protein mixture. Preferably, the basement membrane-like extracellular matrix is the Matrigel® basement membrane matrix (Corning).

The endothelial cells bioprinted under the neuronal spheroids or organoids, or seeded on the basolateral side of the neuronal equivalent, in particular on the basolateral side of the cell culture compartment comprising the neuronal equivalent, resemble the blood-brain-barrier, and its tightness has been shown by a TEER of 20-78 Ω x cm² after two weeks of chip cultivation. Transepithelial/transendothelial electrical resistance (TEER) is a widely accepted quantitative technique to measure the integrity of tight junction dynamics in cell culture models of endothelial and epithelial cell layers. TEER is a strong indicator of the integrity of the cellular barriers before they are evaluated, e.g., for transport of drugs or chemicals. TEER measurements can be performed in real time without cell damage and generally are based on measuring ohmic resistance or measuring impedance across a wide spectrum of frequencies. The measurements for various cell types have been reported with commercially available measurement systems and also with custom-built microfluidic implementations. Some of the barrier models that have been widely characterized using TEER include the blood-brain barrier (BBB), gastrointestinal (GI) tract, and pulmonary models.
In various embodiments of the present disclosure, endothelial cells bioprinted under the neuronal spheroids or organoids, or seeded on the basolateral side of the neuronal equivalent, in particular on the basolateral side of the cell culture compartment comprising the neuronal equivalent, show a TEER of 20-78 Ω x cm², specifically after two weeks of chip cultivation.

In various embodiments of the present disclosure, the cells of the intestinal equivalent are characterized by expression of specific markers of intestinal epithelial cells, including including sucrase-isomaltase, the intestinal oligopeptide transporter SLC15A1/peptide transporter 1 (PEPT1), and the major metabolizing enzyme CYP3A4.

In various embodiments of the present disclosure, the cells of the intestinal equivalent and/or the liver equivalent are characterized by expression of specific markers including the major metabolizing enzyme CYP3A4.

In various embodiments of the present disclosure, the cells of the liver equivalent are characterized by expression of specific markers including cytokeratin 8/18.

In various embodiments of the present disclosure, the liver equivalent is characterized by expression of tight junction ZO-1 protein.

In various embodiments of the present disclosure, the cells of the neuronal equivalent are characterized by expression of specific markers including beta-tubulin III.

In various embodiments of the present disclosure, (primary) endothelial cells are characterized by expression of CD31 and/or von-Willebrand-Faktor (vWF).

In various embodiments of the present disclosure, (iPSC-derived) fibroblasts are characterized by expression of vimentin.

As described herein, any type of cells used and described in the present disclosure may be derived from induced pluripotent stem cells (iPSCs). Specifically, the cells may be derived (*i.e.,* differentiated) from a single (*i.e.,* one and the same) donor iPSC, which in turn has been obtained (*i.e.,* reprogrammed) from a single type of somatic cell. For example, the cells may be derived (*i.e.,* differentiated) from a single (*i.e.,* one and the same) iPSC, which has been obtained (*i.e.,* reprogrammed) from, *e.g.,* a somatic skin cell, white blood cell, renal cell, adipocytes, or the like. Thus, to the extent that the cells are not already identified as iPSC-derived cells, cells used and described in the present disclosure may be iPSC-derived cells according to various embodiments, preferably iPSC-derived cells that have been differentiated from a single (*i.e.,* one and the same) donor iPSC, which has been obtained (*i.e.,* reprogrammed) from a single type of somatic cell.

As described herein, reference to the "intestine" means according to preferred embodiments reference to the "small intestine". For example, reference to "intestine equivalent" or "cells of the intestine" means according to preferred embodiments reference to "small intestine equivalent" or "cells of the small intestine", respectively.

As described herein, reference to "cells" encompasses reference to "mammalian cells", while reference to "human cells" is preferred. For example, reference to "endothelial cells" encompasses reference to "mammalian endothelial cells", while reference to "human endothelial cells" is preferred.

As further described herein, reference to "tissue" encompasses reference to "mammalian tissue", while reference to "human tissue" is preferred. For example, reference to "liver tissue" encompasses reference to "mammalian liver tissue", while reference to "human liver tissue" is preferred.

As further described herein, in various embodiments, the term "tissue" may be considered to be a "tissue culture".

Furthermore, as described herein, "culturing cells" preferably means "fluidic culturing of cells".

In various embodiments, the present disclosure encompasses fluidic (or fluid) shear stress, specifically physiologically relevant fluidic (or fluid) shear stress.

### EXAMPLES

### Chip design of ADME-N Chip

A chip design is realized having constraints scaled down from human physiology. Dimensional data as well as flow characteristics are considered. The layout comprises two circuits (termed "blood" and "urine") containing cavities for the incorporation of the following organ equivalents: intestine, liver, kidney (segregated into glomerulus and tubulus), and neuronal tissue (Fig. 1). The former three tissues are used to accomplish the so-called ADME profile (adsorption, distribution, metabolism, excretion). The latter is an additional tissue supplementing that profile. The chip is, thus, termed ADME-N chip. One reservoir compartment in each circuit allows sampling of supernatants (medium reservoir 1 and 2). The medium is perfused through the microfluidic network by two incorporated, pneumatic micropumps - one for each circuit. The circuits overlap in the two kidney compartments and are separated by a porous membrane made of polycarbonate.

### Intestine compartment

The intestine equivalent was realized by placing a 24-well standing cell culture insert into the respective cavity (cell culture compartment). The insert contained either a commercially available intestinal model (Epilntestinal®, MatTek) or organoids of iPSC-derived intestinal cells embedded into Matrigel containing also iPSC-derived fibroblasts and iPSC-derived endothelial cells. On the iPSC derived intestinal equivalent model, primary microvascular endothelial cells or iPSC-derived endothelial cells were seeded on the basolateral side of the 24-well standing cell culture insert. The apical side of the insert was used for the daily feeding of a nutritious solution (Nutriflex® peri, B.Braun). In total, the apical side contained a supernatant of 250 µL.

### Neuronal compartment

The neuronal equivalent with blood brain barrier consisted of a 96-well hanging cell culture insert containing iPSC-derived neuronal spheroids enriched with iPSC-derived endothelial cells seeded on the basolateral side of the 96-well hanging cell culture insert or bioprinted under the neuronal spheroids. The apical side of the insert contained a volume of 75 µL.

### Liver compartment

The liver equivalent was accomplished either by placing commercially available liver models (3D InSight™ Human Liver Microtissues XL, InSphero) or spheroids of iPSC-derived hepatocytes with iPSC-derived fibroblasts into the respective cavity. Of the former, 50 spheroids were placed into one chip, each containing 3,000 primary hepatocytes. Of the latter, 20 spheroids were placed into one chip, each containing 50,000 cells.

### Kidney (renal) compartment

The kidney compartments were seeded either with a renal proximal tubule epithelial cell line (RPTECs) or with iPSC-derived renal cells.

The used differentiation protocols for the iPSC-derived cells were adapted from different papers listed in Table 1.

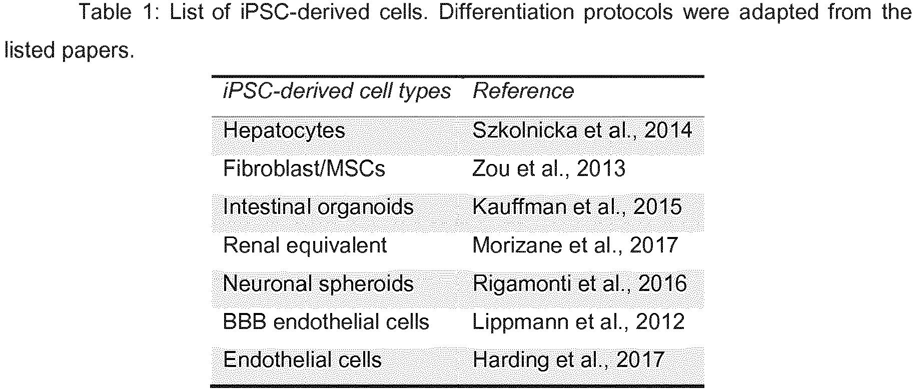

The "blood" circuit had an overall volume of approximately 1.37 mL (not including apical volumes). The "urine" circuit had an overall volume of approximately 0.58 mL. Initially, the chip contained medium with glucose and serum. Every day, samples were taken from both medium reservoirs and from the apical side of the intestine equivalent. The taken volumes were replaced with glucose-free medium for the "blood" medium reservoir and glucose- and serum-free medium for the "urine" medium reservoir. To the intestinal compartment a glucose-rich Nutriflex® peri solution was given. In this way, 1.0 mg of glucose was fed daily to the system only through the intestine equivalent.

Sets of chips were cultivated for 7, 14 and 21 days (Fig. 2). At the endpoints, cellular samples were taken for immunohistological examinations, qPCR and RNA sequencing. Supernatants were analysed for their glucose, lactate, LDH, albumin, urea, ALT and AST content. Controls from static cultivations of the given organ equivalents were taken on day 0, 7 and 14.

### Experiment 1.1: iPSC Chips - 3 chips

- iPSC liver equivalent
- iPSC intestine equivalent
- iPSC neuronal equivalent with blood brain barrier printed
- RPTECs kidney

### Intestine compartment

The insert of the intestine equivalent contains organoids of iPSC-derived intestinal cells embedded into Matrigel containing also iPSC-derived fibroblasts and iPSC-derived endothelial cells. On the iPSC derived intestinal equivalent model, primary microvascular endothelial cells or iPSC-derived endothelial cells are seeded on the basolateral side of the 24-well standing cell culture insert.

### Neuronal compartment

The neuronal equivalent with blood brain barrier consists of a cell culture insert containing iPSC-derived neuronal spheroids enriched with iPSC-derived endothelial cells bioprinted under the neuronal spheroids.

### Liver compartment

The liver equivalent is accomplished by placing spheroids of iPSC-derived hepatocytes with iPSC-derived fibroblasts into the respective cavity. 20 spheroids are placed onto one chip, each containing 50,000 cells (24:1 ratio of hepatocytes:fibroblasts).

### Kidney (renal) compartment

The kidney compartments are seeded with a renal proximal tubule epithelial cell line (RPTECs).

### Experiment 1.2: Primary tissue Chips - 10 chips

- Primary liver tissue
- Primary small intestine
- iPSC neuronal equivalent with blood brain barrier printed
- RPTECs kidney

### Intestine compartment

The insert of the intestine equivalent contains a commercially available intestinal model (Epilntestinal®, MatTek).

### Neuronal compartment

The neuronal equivalent with blood brain barrier consists of a cell culture insert containing iPSC-derived neuronal spheroids enriched with iPSC-derived endothelial cells bioprinted under the neuronal spheroids.

### Liver compartment

The liver equivalent is accomplished by placing commercially available liver models (3D InSight™ Human Liver Microtissues XL, InSphero) into the respective cavity. 50 spheroids are placed onto one chip, each containing 3,000 primary hepatocytes.

### Kidney (renal) compartment

The kidney compartments are seeded with a renal proximal tubule epithelial cell line (RPTECs).

### Experiment 2: iPSC Chips - 21 chips

- iPSC liver equivalent
- iPSC intestine equivalent
- iPSC neuronal equivalent with blood brain barrier
- iPSC renal equivalent

### Intestine compartment

The insert of the intestine equivalent contains organoids of iPSC-derived intestinal cells embedded into Matrigel containing also iPSC-derived fibroblasts and iPSC-derived endothelial cells. On the iPSC derived intestinal equivalent model, primary microvascular endothelial cells or iPSC-derived endothelial cells are seeded on the basolateral side of the 24-well standing cell culture insert.

### Neuronal compartment

The neuronal equivalent with blood brain barrier consists of a cell culture insert containing iPSC-derived neuronal spheroids enriched with iPSC-derived endothelial cells seeded on the basolateral side of the cell culture insert.

### Liver compartment

The liver equivalent is accomplished by placing spheroids of iPSC-derived hepatocytes with iPSC-derived fibroblasts into the respective cavity. 20 spheroids are placed onto one chip, each containing 50,000 cells (24:1 ratio of hepatocytes:fibroblasts).

### Kidney (renal) compartment

The kidney compartments are seeded with iPSC-derived renal cells.

### Results

The chips were homeostatic. This was shown by constant levels of glucose and LDH (Fig. 3). A constant glucose concentration suggested constant consumption and an active regulation of the available glucose. The glucose levels have reached equal levels in all compartments. Feeding only apically through the intestinal equivalent was sufficient to supply both, the "blood" and "urine" circuit. Also, LDH activity has been shown rather constant throughout the experiments. As compared to glucose, LDH levels are different in each compartment. The barrier function of the intestine equivalent and the renal equivalent is shown by different concentrations of LDH in all three compartments *(i.e.,* blood circuit -> medium reservoir 1; urine circuit -> medium reservoir 2; and intestine compartment). The barriers, thus, differentiate between the crossing substances. The active or passive penetration of glucose, while retaining other substances, indicate practical interactions between subsets of the chip system.

The glomerular and tubular compartments were accomplished with a combination of a polycarbonate membrane and a tight layer of renal cells (Fig. 4). The former implements a technical barrier to filter big medium constituents and retards their diffusion. The latter reabsorbs substances and causes the build-up of concentration gradients across the "blood" and "urine" circuit. This gradient is visible not only for LDH but also for aspartate transaminase (AST, Fig. 5). Both their distinctly different concentrations demonstrate the function of the kidney membrane of the ADME-N chip. The AST can only originate from the liver equivalent as it is an intracellular enzyme found in hepatocytes and muscle cells.

All organ equivalents were immunohistochemically assessed to determine identity, functionality and viability (Fig. 6-9). The iPSC-derived intestine equivalent was formed by a layer of Matrigel covered with iPSC-derived fibroblasts. Intestinal organoids enclosing a lumen were incorporated into the gel (Fig. 6). The iPSC-derived intestinal cells expressed NaK-ATPase and Cyp3A4. In Experiment 1.2 a closed layer of primary microvascular endothelial cells was, further, forming another layer on the basolateral side of the cell culture insert.

The primary liver equivalent showed functional markers at the end of its two-week cultivation period (Fig. 7). Also, for the iPSC-derived liver equivalent functional markers were confirmed after three weeks of cultivation in the ADME-N chip (Fig. 8).

The iPSC-derived neuronal equivalent expressed functional markers for β-tubulin III at the end of its two-week cultivation period (Fig. 9). Endothelial cells printed in the hydrogel show vWF expression after two weeks of chip cultivation (Experiment 1.2). The tightness of the blood barrier was shown by TEER values of 20-78 Ω x cm² after two weeks of chip cultivation (Experiment 2).

### List of references cited in Table 1:

1. Szkolnicka, D., Farnworth, S. L., Lucendo-villarin, B. & Hay, D. C. Deriving Functional Hepatocytes from Pluripotent Stem Cells. 1-12 (2014). doi:10.1002/9780470151808.sc01g05s30
2. Zou, L. et al. A simple method for deriving functional MSCs and applied for osteogenesis in 3D scaffolds. Sci. Rep. 3, 2243 (2013).
3. Kauffman, A. L., Ekert, J. E., Gyurdieva, A. V, Rycyzyn, M. A. & Hornby, P. J. Directed differentiation protocols for successful human intestinal organoids derived from multiple induced pluripotent stem cell lines. 2, 1-10 (2015).
4. Morizane, R. & Bonventre, J. V. Generation of nephron progenitor cells and kidney organoids from human pluripotent stem cells. Nat. Protoc. 12, 195-207 (2016).
5. Rigamonti, A. et al. Stem Cell Reports. Stem Cell Reports 6, 993-1008 (2016).
6. Lippmann Ethan S, Azarin Samira M, Kay Jennifer E, Nessler Randy A, Wilson Hannah K, Al-Ahmad Abraham, Palecek Sean P, S. E. V. Human Blood-Brain Barrier Endothelial Cells Derived from Pluripotent Stem Cells. Nat. Biotechnol. 30, 783-791 (2012).
7. Harding, A. et al. Highly Efficient Differentiation of Endothelial Cells from Pluripotent Stem Cells Requires the MAPK and the PI3K Pathways. Stem Cells 35, 909-919 (2017).

## Claims

1. A microfluidic device comprising a blood circuit with one or more cell culture compartments and a urine circuit comprising a filtration unit and a reabsorption unit, wherein both circuits are connected with each other via the filtration unit and the reabsorption unit, and further comprising a reservoir compartment in addition to the one or more cell culture compartments, which serves to add nutrient solution to the system.

2. The microfluidic device of claim 1, wherein the filtration unit comprises a filtration barrier that selectively allows the passage of molecules from the blood circuit to the urine circuit based on size and charge of the molecules.

3. The microfluidic device of claim 2, wherein the filtration barrier is a mechanical or biological barrier, preferably a biological barrier that comprises podocytes.

4. The microfluidic device of any one of claims 1-3, wherein the reabsorption unit comprises a barrier that allows reabsorption of fluid from the urine circuit to the blood circuit.

5. The microfluidic device of claim 4, wherein the reabsorption barrier is a biological barrier, preferably wherein the biological barrier comprises renal tubule cells.

6. The microfluidic device of any one of claims 1-5, wherein at least one of the cell culture compartments of the blood circuit comprises an organ equivalent mimicking liver function, and at least one of the cell culture compartments of the blood circuit comprises an organ equivalent mimicking intestine function.

7. Use of the microfluidic device of any one of claims 1-6 in analytical testing, diagnostics, research, target validation, toxicity studies, tissue engineering, tissue manufacturing, drug screening, and/or pharmacokinetic-pharmacodynamic analysis.

8. A method of operating a microphysiological system making use of the microfluidic device of any one of claims 1-6.

9. The method of claim 8, further comprising selectively adding a nutrient solution to the microphysiological system via the cell culture compartment comprising an organ equivalent or tissue comprising epithelial cells of the gastrointestinal tract and mimicking intestine function.

10. A method of detecting an analyte in a microphysiological system making use of the microfluidic device of any one of claims 1-6, wherein the method comprises adding a test sample to a cell culture compartment of the blood circuit, preferably wherein the cell culture compartment comprises a tissue comprising epithelial cells of the gastrointestinal tract, the skin, or the respiratory tract.

11. A method of mimicking homeostasis comprising operating a microphysiological system making use of the microfluidic device of any one of claims 1-6 comprising a blood circuit with one or more cell culture compartments and a urine circuit comprising a filtration unit and a reabsorption unit, wherein both circuits are connected with each other via the filtration unit and the reabsorption unit.

12. The method of claim 11, comprising selectively adding a nutrient solution to the microphysiological system via a cell culture compartment comprising epithelial cells of the gastrointestinal tract, the skin, or the respiratory tract, preferably wherein the epithelial cells are epithelial cells of the gastrointestinal tract, preferably of the small intestine.

13. A method of culturing and/or maintaining cells comprising seeding the microfluidic device of any one of claims 1-6 with cells.

14. A kit for operating a microphysiological system comprising the microfluidic device of any one of claims 1-6 and instructions for use.

15. An ADME (absorption, distribution, metabolism, excretion) assay, or an ADMET (absorption, distribution, metabolism, excretion, toxicity) assay, comprising the microfluidic device according to any one of claims 1-6.

## Patentansprüche

1. Mikrofluidische Vorrichtung, umfassend einen Blutkreislauf mit einem oder mehreren Zellkulturkompartimenten und einen Urinkreislauf, welcher eine Filtrationseinheit und eine Reabsorptionseinheit umfasst, wobei beide Kreisläufe über die Filtrationseinheit und die Reabsorptionseinheit miteinander verbunden sind, und weiterhin umfassend ein Reservoir-Kompartiment zusätzlich zu dem einen oder mehreren Zellkulturkompartimenten, welches dazu dient, dem System Nährlösung zuzuführen.

2. Die mikrofluidische Vorrichtung nach Anspruch 1, wobei die Filtrationseinheit eine Filtrationsbarriere umfasst, welche selektiv den Durchgang von Molekülen aus dem Blutkreislauf in den Urinkreislauf basierend auf Größe und Ladung der Moleküle erlaubt.

3. Die mikrofluidische Vorrichtung nach Anspruch 2, wobei es sich bei der Filtrationsbarriere um eine mechanische oder biologische Barriere handelt, vorzugsweise um eine biologische Barriere, welche Podozyten umfasst.

4. Die mikrofluidische Vorrichtung nach einem der Ansprüche 1 bis 3, wobei die Reabsorptionseinheit eine Barriere umfasst, welche die Reabsorption von Flüssigkeit aus dem Urinkreislauf in den Blutkreislauf erlaubt.

5. Die mikrofluidische Vorrichtung nach Anspruch 4, wobei es sich bei der Reabsorptionsbarriere um eine biologische Barriere handelt, wobei die biologische Barriere vorzugsweise Nierentubuluszellen umfasst.

6. Die mikrofluidische Vorrichtung nach einem der Ansprüche 1 bis 5, wobei mindestens eines der Zellkulturkompartimente des Blutkreislaufs ein Organäquivalent umfasst, welches Leberfunktion nachahmt, und mindestens eines der Zellkulturkompartimente des Blutkreislaufs ein Organäquivalent umfasst, welches Darmfunktion nachahmt.

7. Verwendung der mikrofluidischen Vorrichtung nach einem der Ansprüche 1 bis 6 bei analytischen Tests, in der Diagnostik, Forschung, Target-Validierung, bei Toxizitätsstudien, Tissue Engineering, Gewebeherstellung, Arzneimittel-Screening, und/oder bei der pharmakokinetisch-pharmakodynamischen Analyse.

8. Verfahren zum Betreiben eines mikrophysiologischen Systems unter Verwendung der mikrofluidischen Vorrichtung nach einem der Ansprüche 1 bis 6.

9. Verfahren nach Anspruch 8, weiterhin umfassend die selektive Zugabe einer Nährlösung zu dem mikrophysiologischen System über das Zellkulturkompartiment, welches ein Organäquivalent oder Gewebe umfasst, das Epithelzellen des Gastrointestinaltrakts umfasst und Darmfunktion nachahmt.

10. Verfahren zum Nachweis eines Analyten in einem mikrophysiologischen System unter Verwendung der mikrofluidischen Vorrichtung nach einem der Ansprüche 1 bis 6, wobei das Verfahren die Zugabe einer Testprobe zu einem Zellkulturkompartiment des Blutkreislaufs umfasst, wobei das Zellkulturkompartiment vorzugsweise ein Gewebe umfasst, welches Epithelzellen des Gastrointestinaltrakts, der Haut, oder der Atemwege umfasst.

11. Verfahren zur Nachahmung von Homöostase, umfassend das Betreiben eines mikrophysiologischen Systems unter Verwendung der mikrofluidischen Vorrichtung nach einem der Ansprüche 1 bis 6, umfassend einen Blutkreislauf mit einem oder mehreren Zellkulturkompartimenten und einen Urinkreislauf mit einer Filtrationseinheit und einer Reabsorptionseinheit, wobei beide Kreisläufe über die Filtrationseinheit und die Reabsorptionseinheit miteinander verbunden sind.

12. Verfahren nach Anspruch 11, umfassend die selektive Zugabe einer Nährlösung zu dem mikrophysiologischen System über ein Zellkulturkompartiment, welches Epithelzellen des Gastrointestinaltrakts, der Haut, oder der Atemwege umfasst, wobei es sich bei den Epithelzellen vorzugsweise um Epithelzellen des Gastrointestinaltrakts, vorzugsweise des Dünndarms, handelt.

13. Verfahren zum Züchten und/oder Aufrechterhalten von Zellen, umfassend das Besetzen der mikrofluidischen Vorrichtung nach einem der Ansprüche 1 bis 6 mit Zellen.

14. Kit zum Betreiben eines mikrophysiologischen Systems, umfassend die mikrofluidische Vorrichtung nach einem der Ansprüche 1-6 und eine Gebrauchsanweisung.

15. ADME-Assay (Absorption, Distribution, Metabolismus, Exkretion) oder ADMET-Assay (Absorption, Distribution, Metabolismus, Exkretion, Toxizität), umfassend die mikrofluidische Vorrichtung nach einem der Ansprüche 1 bis 6.

## Revendications

1. Dispositif microfluidique comprenant un circuit sanguin comportant un ou plusieurs compartiments de culture cellulaire et un circuit urinaire comprenant une unité de filtration et une unité de réabsorption, les deux circuits étant reliés l'un à l'autre par l'intermédiaire de l'unité de filtration et de l'unité de réabsorption, et comprenant en outre un compartiment réservoir en plus du ou des compartiments de culture cellulaire, qui sert à ajouter de la solution nutritive dans le système.

2. Dispositif microfluidique selon la revendication 1, dans lequel l'unité de filtration comprend une barrière filtrante qui autorise sélectivement le passage de molécules du circuit sanguin au circuit urinaire sur la base de la taille et de la charge des molécules.

3. Dispositif microfluidique selon la revendication 2, dans lequel la barrière filtrante est une barrière mécanique ou biologique, de préférence une barrière biologique qui comprend des podocytes.

4. Dispositif microfluidique selon l'une quelconque des revendications 1 à 3, dans lequel l'unité de réabsorption comprend une barrière qui autorise la réabsorption de fluide du circuit urinaire vers le circuit sanguin.

5. Dispositif microfluidique selon la revendication 4, dans lequel la barrière de réabsorption est une barrière biologique, la barrière biologique comprenant de préférence des cellules de tubules rénaux.

6. Dispositif microfluidique selon l'une quelconque des revendications 1 à 5, dans lequel l'un au moins des compartiments de culture cellulaire du circuit sanguin comprend un équivalent d'organe imitant la fonction hépatique, et l'un au moins des compartiments de culture cellulaire du circuit sanguin comprend un équivalent d'organe imitant la fonction intestinale.

7. Utilisation d'un dispositif microfluidique selon l'une quelconque des revendications 1 à 6 en essais analytiques, diagnostic, recherche, validation de cible, études de toxicité, ingénierie tissulaire, fabrication de tissus, criblage de médicaments, et/ou analyse pharmacocinétique-pharmacodynamique.

8. Procédé de fonctionnement d'un système microphysiologique utilisant le dispositif microfluidique selon l'une quelconque des revendications 1 à 6.

9. Procédé selon la revendication 8, comprenant en outre l'ajout sélectif d'une solution nutritive au système microphysiologique par l'intermédiaire du compartiment de culture cellulaire comprenant un équivalent d'organe ou un tissu comprenant des cellules épithéliales du tractus gastro-intestinal et imitant la fonction intestinale.

10. Procédé de détection d'un analyte dans un système microphysiologique utilisant le dispositif microfluidique selon l'une quelconque des revendications 1 à 6, le procédé comprenant l'ajout d'un échantillon pour essai dans un compartiment de culture cellulaire du circuit sanguin, le compartiment de culture cellulaire comprenant de préférence un tissu comprenant des cellules épithéliales du tractus gastro-intestinal, de la peau, ou des voies respiratoires.

11. Procédé imitant l'homéostasie comprenant l'exploitation d'un système microphysiologique utilisant le dispositif microfluidique selon l'une quelconque des revendications 1 à 6 comprenant un circuit sanguin comportant un ou plusieurs compartiments de culture cellulaire et un circuit urinaire comprenant une unité de filtration et une unité de réabsorption, les deux circuits étant reliés l'un à l'autre par l'intermédiaire de l'unité de filtration et de l'unité de réabsorption.

12. Procédé selon la revendication 11, comprenant l'ajout sélectif d'une solution nutritive au système microphysiologique par l'intermédiaire d'un compartiment de culture cellulaire comprenant cellules épithéliales du tractus gastro-intestinal, de la peau, ou des voies respiratoires, les cellules épithéliales étant de préférence des cellules épithéliales du tractus gastro-intestinal, de préférence de l'intestin grêle.

13. Procédé de culture et/ou de maintien de cellules comprenant l'ensemencement du dispositif microfluidique selon l'une quelconque des revendications 1 à 6 avec des cellules.

14. Kit d'exploitation d'un système microphysiologique comprenant le dispositif microfluidique selon l'une quelconque des revendications 1 à 6 et des instructions d'utilisation.

15. Essai ADME (absorption, distribution, métabolisme, excrétion), ou essai ADMET (absorption, distribution, métabolisme, excrétion, toxicité), comprenant le dispositif microfluidique selon l'une quelconque des revendications 1 à 6.
